# EUROPEAN PATENT SPECIFICATION

(11) **EP 2 437 767 B1**
(45) Date of publication and mention of the grant of the patent: **08.07.2015**
(21) Application number: 10783845.0
(22) Date of filing: 28.05.2010
(51) Int. Cl.: C07K 19/00, A61K 47/48, C07K 14/315, C07K 16/00

(54) **MOLECULES WITH EXTENDED HALF-LIVES AND USES THEREOF**
MOLEKÜLE MIT VERLÄNGERTER HALBWERTZEIT UND VERWENDUNGEN DAFÜR
MOLÉCULES AYANT UNE DEMI-VIE PROLONGÉE ET LEURS UTILISATIONS

(30) Priority: 01.06.2009 US 182858 P
(43) Date of publication of application: 11.04.2012
(73) Proprietor: MedImmune, LLC, Gaithersburg, MD 20878 (US)
(72) Inventor: LUBMAN, Olga, Saint Louis MO 63108 (US); DALL'ACQUA, William, Gaithersburg MD 20878 (US); WU, Herren, Boyds MD 20841 (US)
(74) Representative: Winter, Christopher Spencer
(86) International application number: PCT/US2010/036497
(87) International publication number: WO 2010/141329

(56) References cited:
- WO-A1-2009/040562
- US-A1- 2007 071 764
- US-A1- 2008 267 949
- US-A1- 2009 074 770
- WINGREN C ET AL: "Surface properties of antigen-antibody complexes.", SCANDINAVIAN JOURNAL OF IMMUNOLOGY AUG 1997, vol. 46, no. 2, August 1997 (1997-08), pages 159-167, XP002692542, ISSN: 0300-9475
- STORK R ET AL: "A novel tri-functional antibody fusion protein with improved pharmacokinetic properties generated by fusing a bispecific single-chain diabody with an albumin-binding domain from streptococcal protein G", PROTEIN ENGINEERING, DESIGN AND SELECTION, OXFORD JOURNAL, LONDON, GB, vol. 20, no. 11, 1 November 2007 (2007-11-01), pages 569-576, XP002515447, ISSN: 1741-0126, DOI: 10.1093/PROTEIN/GZM061 [retrieved on 2007-11-03]
- J. T. ANDERSEN ET AL: "Extending Half-life by Indirect Targeting of the Neonatal Fc Receptor (FcRn) Using a Minimal Albumin Binding Domain", JOURNAL OF BIOLOGICAL CHEMISTRY, vol. 286, no. 7, 18 February 2011 (2011-02-18), pages 5234-5241, XP055019819, ISSN: 0021-9258, DOI: 10.1074/jbc.M110.164848
- CARTER, P.J.: 'Potent antibody therapeutics by design' NATURE REVIEWS: IMMUNOLOGY vol. 6, May 2006, pages 343 - 357, XP007901440

## Description

### FIELD OF THE INVENTION

The present invention provides a means of increasing the half-life of a bioactive agent of interest. Specifically, the bioactive agent is associated with a molecule that combines an albumin binding domain and an FcRn binding IgG Fc fragment. The advantage of increasing the half-life of a bioactive agent of interest is that smaller amounts and/or less frequent dosing is required in the therapeutic, prophylactic or diagnostic use of such bioactive agents.

### BACKGROUND OF THE INVENTION

The use of immunoglobulins as therapeutic agents has increased dramatically in recent years and has expanded into different areas of medical treatments. Such uses include treatment of agammaglobulinemia and hypogammaglobulinemia, as immunosuppressive agents for treating autoimmune diseases and graft-vs.-host (GVH) diseases, the treatment of lymphoid malignancies, and passive immunotherapies for the treatment of various systemic and infectious diseases. Also, immunoglobulins are useful as *in vivo* diagnostic tools, for example, in diagnostic imaging procedures.

One critical issue in these therapies is the persistence of immunoglobulins in the circulation. The rate of immunoglobulin clearance directly affects the amount and frequency of dosage of the immunoglobulin. Increased dosage and frequency of dosage may cause adverse effects in the patient and also an increase in medical costs.

Clearance of IgGs is believed to be controlled by portions of the IgG constant domain. The IgG constant domain controls IgG metabolism including the rate of IgG degradation in the serum through interactions with FcRn. Indeed, increased binding affinity for FcRn increased the serum half-life of the molecule (Kim et al., Eur. J. Immunol., 24:2429-2434, 1994; Popov et al, Mol. Immunol., 33:493-502, 1996; Ghetie et al, Eur. J. Immunol., 26:690-696, 1996; Junghans et al., Proc. Natl. Acad. Sci. USA, 93:5512-5516, 1996; Israel et al, Immunol., 89:573-578, 1996).

Various site-specific mutagenesis experiments in the Fc region of mouse IgGs have led to identification of certain critical amino acid residues involved in the interaction between IgG and FcRn (Kim et al, Eur. J. Immunol., 24:2429-2434, 1994; Medesan et al, Eur. J. Immunol., 26:2533, 1996; Medesan et al., J. Immunol., 158:2211-2217, 1997). These studies and sequence comparison studies found that isoleucine at position 253, histidine at position 310, and histidine at position 435 (according to Kabat EU numbering, which refers to the EU index numbering of the human IgG1 Kabat antibody as set forth in Kabat et al., In: Sequences of Proteins of Immunological Interest, US Department of Health and Human Services, 1991 ), are highly conserved in human and rodent IgGs, suggesting their importance in IgG-FcRn binding. Additionally, various publications describe methods for obtaining physiologically active molecules whose half-lives are modified either by introducing an FcRn-binding polypeptide into the molecules (WO 97/43316; U.S. Pat. No. 5,869,046; U.S. Pat. No. 5,747,035; WO 96/32478; WO 91/14438) or by fusing the molecules with antibodies whose FcRn-binding affinities are preserved but affinities for other Fc receptors have been greatly reduced (WO 99/43713) or fusing with FcRn binding domains of antibodies (WO 00/09560; U.S. Pat. No. 4,703,039).

Stork et al., (Protein Engineereing, Design and Selection, 20(11):579-576, 2007) discloses the use of an antigen binding domain from Protein G of Streptococcus that is fused to a bispecific single-chain diabody in order to improve the pharmacokinetic properties thereof.

WO2009040562 discloses the fusion of an anti-human serum albumin single-domain antibody to a Fab-domain in order to improve the half-life thereof.

In view of the pharmaceutical importance of increasing the *in vivo* half-life of a bioactive agent, there is a need to develop a molecule to which the bioactive agent can be associated with in order to confer increased *in* vivo half-life on the bioactive agent of interest.

### SUMMARY OF THE INVENTION

The present invention is directed to extending the pharmacologic properties (including increasing the half-life) of a bioactive agent of interest. This is achieved by associating the bioactive agent, e.g., by genetically linking, chemically fusing or conjugating the bioactive agent, to a molecule that can bind both human serum albumin and the FcRn receptor.

In one aspect the invention includes a molecule comprising an albumin binding domain (ABD) from Streptococcus protein G fused to an FcRn binding IgG Fc fragment, wherein the molecule comprising the ABD fused to an the FcRn binding IgG Fc fragment has a longer half-life than a molecule comprising the the FcRn binding IgG Fc fragment without an ABD or comprising the ABD without the FcRn binding IgG Fc fragment.

The FcRn binding Ig Fc fragment can be an IgG1, IgG2, IgG3, or IgG4 fragment. In one embodiment, the Fc fragment includes a CH2 domain and a CH3 domain. In another embodiment, the Fc fragment includes a hinge region, a CH2 domain and a CH3 domain.

The ABD domain is from Streptococci G protein. In another embodiment, the ABD has an amino acid sequence of SEQ ID NO: 1, or a variant thereof. In another example, the ABD domain has an amino acid sequence of SEQ ID NO:2, or a variant thereof.

In other embodiments, the molecule of the present invention can further include a bioactive agent of interest. In one embodiment, the bioactive agent is a polypeptide such as an antibody.

In another aspect, the invention further includes compositions including the molecules of the present invention. The compositions can include a pharmaceutically acceptable carrier, adjuvant or diluent.

In another aspect, the invention includes a nucleic acid sequence that encodes a molecule of the present invention. In yet other aspects, the invention further includes a host cell having a nucleic acid sequence that encodes a molecule of the present invention.

In another aspect, the invention includes a method of producing a molecule of the present invention. In one embodiment, the method includes culturing a cell line transfected with a nucleic acid that encodes a molecule of the present invention and purifying the polypeptide encoded thereby.

In another aspect, the invention includes a method of increasing the half-life of a bioactive agent of interest comprising fusing the bioactive agent to a molecule having an ABD-FcRn binding IgG Fc fragment. The invention further includes administering the molecule to a mammal such as a primate, e.g., a human.

### BRIEF DESCRIPTION OF THE DRAWINGS

**Fig. 1** shows a schematic of how the ABD-Fc fusion of the present invention binds both Fc and albumin binding sites on cells expressing FcRn and how both sites are believed to be used to recycle the molecule.
**Fig. 2** shows a schematic of the various ABD-Fc Fusion constructs.
**Fig. 3** is a line graph showing pharmacokinetic study results of the ABD-Fc variants.

### TERMINOLOGY

The term "ABD-Fc" as used herein refers to a molecule that has an albumin binding domain from Streptococci G protein (ABD) associated with an FcRn binding IgG Fc fragment (Fc). The term ABD-Fc is not indicative of any particular preference of how the molecule should be assembled. For example, the ABD domain can be at the 5' (i.e.: amino terminal) end of the molecule or can be at the 3' (i.e.: carboxyl terminal) end of the molecule. Moreover, the ABD can be directly or indirectly (e.g., via a linker) associated with the Fc.

The term "ABD-Fc-bioactive agent" refers to a bioactive agent that is associated with the "ABD-Fc" (referred to above) and is not indicative of any particular preference of how the molecule should be assembled. For example, the bioactive agent can be at the 5' (i.e., amino terminal) end of the ABD-Fc molecule or can be at the 3' (i.e., carboxyl terminal) end of the ABD-Fc molecule.

The term "IgG Fc region" as used herein refers the portion of an IgG molecule that correlates to a crystallizable fragment obtained by papain digestion of an IgG molecule. The Fc region consists of the C-terminal half of the two heavy chains of an IgG molecule that are linked by disulfide bonds. It has no antigen binding activity but contains the carbohydrate moiety and the binding sites for complement and Fc receptors, including the FcRn receptor (see below). The Fc fragment contains the entire second constant domain CH2 (residues 231-340 of human IgG1, according to the Kabat EU numbering system) and the third constant domain CH3 (residues 341-447). The sequences are presented herein (see, e.g., SEQ ID NOs: 8-11) and are also disclosed in US 20070122403. The present invention also encompasses IgG Fc regions from different human allotypes. For example, the non-A-allotype of human IgG1 and the A-allotype which have differences in amino acid sequence at positions 356 and 358; additional IgG allotypes are provided for example in Genbank Accession numbers AL928742, AJ390254, AJ390276, AJ390247, J390242, AJ390262, J390272, AJ390241, AJ390237, X16110, AJ390254, and AJ3902725.

The term "IgG hinge-Fc region" or "hinge-Fc fragment" as used herein refers to a region of an IgG molecule consisting of the Fc region (residues 231-447) and a hinge region (residues 216-230) extending from the N-terminus of the Fc region. An example of the amino acid sequence of the human IgG1 hinge-Fc region is presented herein as SEQ ID NO: 8, and is disclosed in US 20070122403.

The term "constant domain" refers to the portion of an immunoglobulin molecule having a more conserved amino acid sequence relative to the other portion of the immunoglobulin, the variable domain, which contains the antigen binding site. The constant domain contains the CH1, CH2 and CH3 domains of the heavy chain and the CHL domain of the light chain.

The term "FcRn receptor" or "FcRn" as used herein refers to an Fc receptor ("n" indicates neonatal) which is known to be involved in transfer of maternal IgGs to a fetus through the human or primate placenta, or yolk sac (rabbits) and to a neonate from the colostrum through the small intestine. It is also known that FcRn is involved in the maintenance of constant serum IgG levels by binding the IgG molecules and recycling them into the serum. The binding of FcRn to IgG molecules is strictly pH-dependent with optimum binding at pH 6.0. FcRn comprises a heterodimer of two polypeptides, whose molecular weights are approximately 50 kD and 15 kD, respectively. The extracellular domains of the 50 kD polypeptide are related to major histocompatibility complex (MHC) class I α-chains and the 15 kD polypeptide was shown to be the non-polymorphic ß2-microglobulin (ß2-microglobulin). In addition to placenta and neonatal intestine, FcRn is also expressed in various tissues across species as well as various types of endothelial cell lines. It is also expressed in human adult vascular endothelium, muscle vasculature and hepatic sinusoids and it is suggested that the endothelial cells may be most responsible for the maintenance of serum IgG levels in humans and mice. The amino acid sequences of human FcRn and murine FcRn are known in the art (e.g., as disclosed in US 20070122403) ). Homologs of these sequences having FcRn activity are also included.

The term "*in vivo* half-life" as used herein refers to a biological half-life of a particular type of molecule or its fragments containing an FcRn-binding site in the circulation of a given animal and is represented by a time required for half the quantity administered in the animal to be cleared from the circulation and/or other tissues in the animal. When a clearance curve of a given IgG is constructed as a function of time, the curve is usually biphasic with a rapid alpha-phase which represents an equilibration of the injected IgG molecules between the intra- and extra-vascular space and which is, in part, determined by the size of molecules, and a longer beta-phase which represents the catabolism of the IgG molecules in the intravascular space. The term "in vivo half-life" practically corresponds to the half life of the IgG molecules in the beta-phase.

An "isolated" or "purified" molecule of the present invention is substantially free of cellular material or other contaminating proteins from the cell or tissue source from which the protein is derived, or substantially free of chemical precursors or other chemicals when chemically synthesized. The language "substantially free of cellular material" includes preparations of ABD-Fc or ABD-Fc-bioactive agent which is separated from cellular components of the cells from which it is isolated or recombinantly produced. Thus, an ABD-Fc or ABD-Fc-bioactive agent that is substantially free of cellular material includes preparations of ABD-Fc or ABD-Fc-bioactive agent having less than about 30%, 20%, 10%, or 5% (by dry weight) of contaminating protein. When the ABD-Fc or ABD-Fc-bioactive agent is recombinantly produced, it is also preferably substantially free of culture medium, i.e., culture medium represents less than about 20%, 10%, or 5% of the volume of the protein preparation. When the ABD-Fc or ABD-Fc-bioactive agent is produced by chemical synthesis, it is preferably substantially free of chemical precursors or other chemicals, i.e., it is separated from chemical precursors or other chemicals which are involved in the synthesis of the protein. Accordingly such preparations of the ABD-Fc or ABD-Fc-bioactive agent have less than about 30%, 20%, 10%, 5% (by dry weight) of chemical precursors or compounds other than ABD-Fc or ABD-Fc-bioactive agent.

An "isolated" nucleic acid molecule is one which is separated from other nucleic acid molecules which are present in the natural source of the nucleic acid molecule. Moreover, an "isolated" nucleic acid molecule, such as a cDNA molecule, can be substantially free of other cellular material, or culture medium when produced by recombinant techniques, or substantially free of chemical precursors or other chemicals when chemically synthesized. An "isolated" nucleic acid molecule does not include cDNA molecules within a cDNA library. In a preferred embodiment of the invention, nucleic acid molecules encoding antibodies are isolated or purified. In another preferred embodiment of the invention, nucleic acid molecules encoding ABD-Fc or ABD-Fc-bioactive agents are isolated or purified.

The term "host cell" as used herein refers to the particular subject cell transfected with a nucleic acid molecule or infected with phagemid or bacteriophage and the progeny or potential progeny of such a cell. Progeny of such a cell may not be identical to the parent cell transfected with the nucleic acid molecule due to mutations or environmental influences that may occur in succeeding generations or integration of the nucleic acid molecule into the host cell genome.

The names of amino acids referred to herein are abbreviated either with three-letter or one-letter symbols.

To determine the percent identity of two amino acid sequences or of two nucleic acid sequences, the sequences are aligned for optimal comparison purposes (e.g., gaps can be introduced in the sequence of a first amino acid or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity=number of identical overlapping positions/total number of positions x 100%). In one embodiment, the two sequences are the same length.

The determination of percent identity between two sequences can also be accomplished using a mathematical algorithm. A preferred, non-limiting example of a mathematical algorithm utilized for the comparison of two sequences is the algorithm of Karlin and Altschul, 1990, Proc. Natl. Acad. Sci. U.S.A. 87:2264-2268, modified as in Karlin and Altschul, 1993, Proc. Natl. Acad. Sci. U.S.A. 90:5873-5877. Such an algorithm is incorporated into the NBLAST and XBLAST programs of Altschul et al., 1990, J. Mol. Biol. 215:403. BLAST nucleotide searches can be performed with the NBLAST nucleotide program parameters set, e.g., for score=100, wordlength=12 to obtain nucleotide sequences homologous to a nucleic acid molecules of the present invention. BLAST protein searches can be performed with the XBLAST program parameters set, e.g., to score-50, wordlength=3 to obtain amino acid sequences homologous to a protein molecule of the present invention. To obtain gapped alignments for comparison purposes, Gapped BLAST can be utilized as described in Altschul et al., 1997, Nucleic Acids Res. 25:3389-3402. Alternatively, PSI-BLAST can be used to perform an iterated search which detects distant relationships between molecules (Id.). When utilizing BLAST, Gapped BLAST, and PSI-Blast programs, the default parameters of the respective programs (e.g., of XBLAST and NBLAST) can be used (see, e.g., http://www.ncbi.nlm.nih.gov). Another preferred, non-limiting example of a mathematical algorithm utilized for the comparison of sequences is the algorithm of Myers and Miller, 1988, CABIOS 4:11-17. Such an algorithm is incorporated in the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The percent identity between two sequences can be determined using techniques similar to those described above, with or without allowing gaps. In calculating percent identity, typically only exact matches are counted.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

### DETAILED DESCRIPTION

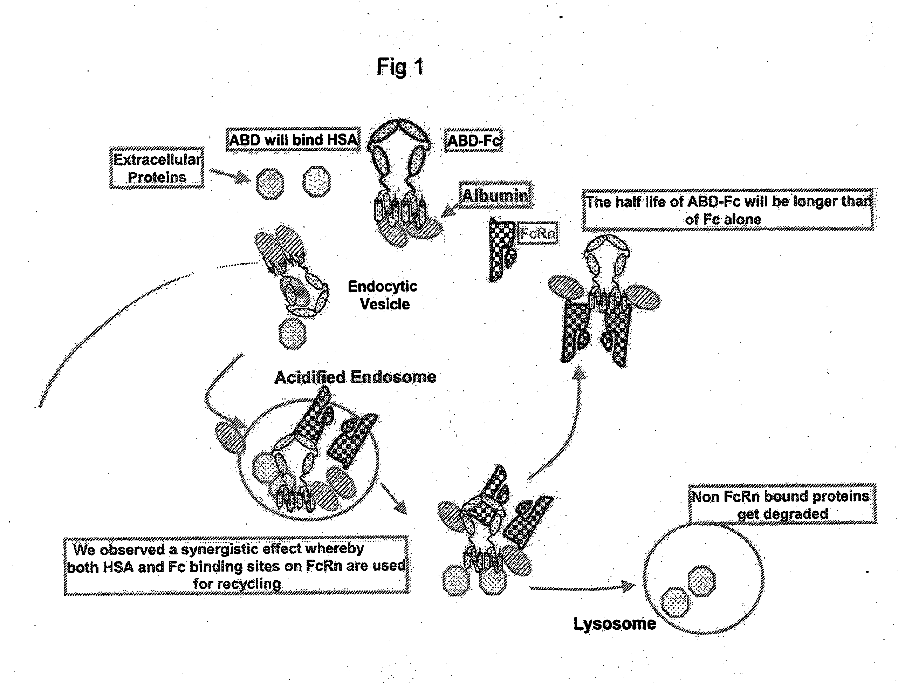

The invention is based, in part, on associating a bioactive agent of interest with a molecule that combines the long half-life of an antibody and albumin in one so as to enhance the pharmacokinetic properties of that bioactive agent. Specifically, this pharmacokinetic enhancing molecule of the invention includes an albumin binding domain from Streptococci G protein (ABD) and an FcRn binding IgG Fc fragment and is referred to hereafter as the "ABD-Fc molecule". Figure 1 shows a schematic of how the ABD-Fc molecule of the present invention can bind albumin and thereafter engage with both Fc and albumin binding sites on FcRn. It is believed that the enhanced half-life of the ABD-Fc molecule is achieved because both sites on FcRn are used in recycling.

### Albumin Binding Domain (ABD)

The ABD is be derived from *Streptococcus*,. ABD domains are described in Johansson et al. (The Journal of Biological Chemistry, 277:8114-8120, 2002).

Typically, the ABD domain of the present invention is small, for example, approximately 70, 60, 50, 49, 48, 47, 46, 45, 44, 43, 42, 41, 40, 39, 38, 37,36, 35, 34, 33, 32, 31, 30, 28, 27, or 26 amino acids in length and can bind albumin such as human serum albumin. For example, the ABD domain can bind albumin at an affinity of 1 mM to 1 nM. Accordingly, the ABD domain of the present invention derived from a protein capable of binding human serum albumin (e.g., streptococcal protein G) will comprise at least that portion sufficient for binding human serum albumin (e.g., SEQ ID NO: 1), but will not comprise the entire amino acid sequence of the protein. The domain can further include a triple-helix bundle such as a left-handed triple helix bundle. In certain embodiments, the ABD domain comprises SEQ ID NO: 1 or SEQ ID NO: 2. In other embodiments, the ABD domain consists of SEQ ID NO: 1 or SEQ ID NO: 2.

The disclosure also includes variants of known ABD domains. ABD variants can contain at least one amino acid alteration such as a conservative amino acid substitution compared to the amino acid sequence of the wild-type ABD or can include deletions of amino acids from the domain. ABD variants can have at least 99%, 98%, 97%, 96%, 95%, 95%, 94%, 93%, 92%, 91%, 90%, 85%, 80%, 75%, 70%, or 65% sequence identity with the wildtype ABD.

ABD variants typically have the same activity or substantially the same activity as wild-type ABDs. For example, the activity can be the ability of the domain to bind albumin, e.g., the variant can bind albumin with an affinity (KD) of at least 1mM. ABD variants also include fragments of ABDs which retain the ability to bind albumin. The fragment can be an ABD domain lacking, e.g., 1, 2, 3, 4, 5, 6, 7, 8, 9, 10, 11, 12, 13, 14, 15 or more amino acids.

Examples of ABDs of the present invention include:
G148-GA3: LAEAKVLANRELDKYGVSDYYKNLINNAKTVEGVKALIDEILAALP (SEQ ID NO:1)
ALB8-GA: LKNAKEDAIAELKKAGITSDFYFNAINKAKTVEEVNALKNEILKA (SEQ ID NO:2)

It will be appreciated that variants of G148-GA3 and ALB8-GA can have at least 99%, 98%, 97%, 96%, 95%, 95%, 94%, 93%, 92%, 91%, 90%, 85%, 80%, 75%, 70%, or 65% sequence identity with SEQ ID NO:1 or SEQ ID NO:2 and retain the ability to bind human serum albumin.

Variants of G148-GA3 and ALB8-GA can also include ABDs having amino acid substitutions, deletions, or insertions of one or more amino acids. In one example, the ABD has 1, 2, 3, 4, 5, 6, 7, 8, 9, 10,20 or more conservative amino acid substitutions.

In one example, the disclosure includes a variant of G148-GA3. The G148-GA3 variant should have substantially the same activity as G148-GA3 and bind human serum albumin with around the same affinity. Furthermore the G148-GA3 should exhibit a left-handed triple helix bundle conformation. Residues critical for binding albumin are present in helix 2 and include residues S 18, Y20, and Y21 and residues critical to the formation of the left-handed triple helix bundle include L12, V33, 1I37 and I40. (Critical residues are bolded and underlined below.) The critical residues may be less tolerant of non-conservative amino acid substitutions, accordingly, maintaining these is generally preferred. However, conservative substitutions may be utilized at critical residues.
LAEAKVLANRE**L**DKYGV**S**D**YY**KNLINNAKTVEG**V**KAL**I**DE**I**LAALP (SEQ ID NO:1)

Thus a variant of G148-GA3 should retain the critical amino acids and can include one or more amino acid substitutions otherwise along the length of SEQ ID NO:1 or be a fragment of SEQ ID NO:1.

Variants of the ABD domain can be tested for their ability to bind human serum albumin (for example as outlined in Example 2). The variants can be further tested to determine if they can bind the FcRn (for example, as outlined in Example 3)

The invention further includes the nucleic acid sequences that encode the ABDs of the invention. Due to the inherent degeneracy of the genetic code, other DNA sequences which encode substantially the same or a functionality equivalent amino acid sequence, may be used to clone and express the ABD domain.

### FCRn binding IgG Fc fragment

The ABD further includes a constant region of an Ig molecule or a region thereof that can bind the FcRn. In one example, the ABD can be fused to the constant heavy chain regions of an IgG antibody. For example, the ABD domain can be linked to the CH2 and CH3 domains of a human Ig molecule or the hinge, CH2 and CH3 domains of the human IgG molecule. In other examples the ABD is linked to one or more of the following sequences: HQNLSDGK (SEQ ID NO:12); HQNISDGK (SEQ ID NO:13) ; VISSHLGQ (SEQ ID NO:14) ; PKNSSMISNTP (SEQ ID NO: 15) to facilitate binding to the FcRn (see, for example US 5,869,046). Any isoform of an Ig molecule can be used to generate molecules according to the present invention, such as isoforms IgG1, IgG2, IgG3 or IgG4, or other Ig classes, like IgM or IgA.

It can be well appreciated that the FcRn binding IgG Fc fragment might also be different from the wild-type molecules described above and still convey the property of possessing a region that binds the FcRn such that the half-life of a bioactive agent is extended. The FcRn binding IgG Fc fragment can be generated by other means. For example, the FcRn binding IgG Fc fragment can be one that was identified using mutagenesis studies of Fc region from IgG or identified following screening for binding with FcRn using peptide or polypeptide libraries. For example, US20080181887 discloses an Fc region from an IgG that can have an extended half-life when having one or more amino acid modifications are at one or more of positions 251-256, 285-290, 308-314,385-389, and 428-436, according to the Kabat EU numbering system. In a specific example, the Fc region comprises one or more of the following substitutions: M252Y, S254T, T256E, M428T, according to the Kabat EU numbering system, which extend half-life. Moreover FcRn binding IgG Fc fragment of the invention include Fc region variants having altered effector function (e.g., antibody dependent cell-mediated cytotoxicity (ADCC); and complement dependent cytotoxicity (CDC)). For example US 6,737,056 and US20060024298 disclose amino acid modifications that can be made in the Fc region which will alter effector function. In a specific example, the Fc region comprises one or more of the following substitutions: S228P, L234F, L235E, L235F, 235Y, P331, wherein the numbering is according to the Kabat EU numbering, which reduced effector functions. Alternatively, FcRn binders can be made using phage display (Ghetie et al. 1997 Nat. Biotechnol. 15(7):637-40.; Dall'Acqua et al. 2002 J. Immunol; 169(9):5171-80 ; WO 2007/098420).

### ABD-FcRn binding IgG Fc fragment

The ABD and the FcRn binding IgG Fc fragment can be made by any means known in the art. For example, the ABD can be associated with the FcRn binding IgG Fc fragment by genetic fusion or chemical conjugation. In one embodiment the ABD domain is directly fused or conjugated to the FcRn binding IgG Fc fragment. In another embodiment, the ABD domain is indirectly fused or conjugated to the FcRn binding IgG Fc fragment, e.g. via a linker peptide sequence. The ABD-Fc of the invention can be a protein formed by the fusion of an albumin binding domain to an FcRn binding IgG Fc fragment and is not indicative of any particular preference of how the molecule should be assembled. Accordingly, the ABD and FcRn binding IgG Fc fragment may be associated in any order, for example, the ABD domain can be at the 5' (i.e., amino terminal) end of the molecule or can be at the 3' (i.e., carboxyl terminal) end of the molecule.

In one embodiment, the ABD and FcRn are genetically fused. In this example, the ABD and FcRn can be fused recombinantly, i.e., wherein a gene construct encoding the ABD and FcRn binding IgG Fc fragment are introduced into an expression system in a manner that allows correct assembly of the molecule upon expression therefrom. In this manner, the ABD domain can be expressed such that it binds albumin and the Fc region is expressed such that it binds FcRn. In one example, the ABD domain is linked to the Fc region with its hinge, CH2, and CH3 domains of an IgG1/2/3/4.

Another example of an ABD-Fc of the invention is the ABD domain G148-GA3 fused to IgG2aFc. The nucleic acid sequence encoding the G148-GA3-IgG2aFc and amino acid sequences are shown below (G148-GA3 is shown underlined).

In certain aspects an ABD-Fc of the invention comprises the ABD domain G148-GA3 (SEQ ID NO: 1), fused to a human IgG1Fc comprising the CH2 and CH3 domains and optionally the hinge domain. In other aspects an ABD-Fc of the invention comprises the ABD domain ALB8-GA (SEQ ID NO:2), fused to a human IgG1Fc comprising the CH2 and CH3 domains and optionally the hinge domain. The hinge, CH2 and CH3 domains of human IgG1 are shown below (SEQ ID NO: 8), the hinge is shown with a single underline, the CH2 is shown with a double underline, and the CH3 is show with a dashed underline.

In certain aspects an ABD-Fc of the invention comprises the ABD domain G148-GA3 (SEQ ID NO: 1), fused to a human IgG2Fc comprising the CH2 and CH3 domains and optionally the hinge domain. In other aspects an ABD-Fc of the invention comprises the ABD domain ALB8-GA (SEQ ID NO:2), fused to a human IgG2Fc comprising the CH2 and CH3 domains and optionally the hinge domain. The hinge, CH2 and CH3 domains of human IgG2 are shown below (SEQ ID NO: 9), the hinge is shown with a single underline, the CH2 is shown with a double underline, and the CH3 is show with a dashed underline.

In certain aspects an ABD-Fc of the invention comprises the ABD domain G148-GA3 (SEQ ID NO: 1), fused to a human IgG3Fc comprising the CH2 and CH3 domains and optionally the hinge domain. In other aspects an ABD-Fc of the invention comprises the ABD domain ALB8-GA (SEQ ID NO:2), fused to a human IgG3Fc comprising the CH2 and CH3 domains and optionally the hinge domain. The hinge, CH2 and CH3 domains of human IgG3 are shown below (SEQ ID NO: 10), the hinge is shown with a single underline, the CH2 is shown with a double underline, and the CH3 is show with a dashed underline.

In certain aspects an ABD-Fc of the invention comprises the ABD domain G148-GA3 (SEQ ID NO: 1), fused to a human IgG4Fc comprising the CH2 and CH3 domains and optionally the hinge domain. In other aspects an ABD-Fc of the invention comprises the ABD domain ALB8-GA (SEQ ID NO:2), fused to a human IgG4Fc comprising the CH2 and CH3 domains and optionally the hinge domain. The hinge, CH2 and CH3 domains of human IgG4 are shown below (SEQ ID NO: 11), the hinge is shown with a single underline, the CH2 is shown with a double underline, and the CH3 is show with a dashed underline.

### Bioactive agent

The present invention encompasses associating the ABD-Fc to a bioactive agent such as a diagnostic or therapeutic agent or any other molecule for which *in vivo* half-life is desired to be increased. While not wishing to be bound by theory, it is believed that the ABD-Fc molecule can increase the half-life of a molecule to which it is associated because it is degraded at a slower rate than if the bioactive agent was associated with either just albumin or Fc alone. This is believed to occur because both the albumin, which binds to the ABD domain, and FcRn binding IgG Fc fragment can bind cells expressing the FcRn receptor. The ability of both ABD and Fc of the ABD-Fc molecule to bind cells expressing the FcRn receptor is believed to increase the chance of rescue of the ABD-Fc-bioactive molecule from lysosomal degradation and recycle it back to the cell surface thereby conferring on the molecule a surprisingly (synergistic) extended half-life compared to associating the molecule with either just albumin or Fc. In one example, the half-life of the bioactive agent is extended by 10%, 20%, 30%, 40%, 50%, 60%, 70% or more compared if the bioactive agent were associated with just albumin or Fc. In certain embodiments, the half-life of the bioactive agent is extended by at least 10% compared if the bioactive agent were associated with just albumin or Fc. In other embodiments, the half-life of the bioactive agent is extended by at least 20% compared if the bioactive agent were associated with just albumin or Fc. In still other embodiments, the half-life of the bioactive agent is extended by at least 30% compared if the bioactive agent were associated with just albumin or Fc.

The bioactive agent and the ABD-Fc can be made by any means known in the art. For example, the bioactive agent can be associated with the ABD-Fc by genetic fusion or chemical conjugation. In one embodiment the ABD domain is directly fused or conjugated to the FcRn binding IgG Fc fragment. In another embodiment, the the bioactive agent is indirectly fused or conjugated to the ABD-Fc, e.,g via a linker peptide sequence. The ABD-Fc-bioactive agent of the invention can be a protein formed by the fusion of an bioactive agent to an ABD-Fc and is not indicative of any particular preference of how the molecule should be assembled. Accordingly, the ABD-FcR may be associated in any order, for example, the ABD domain can be at the 5' (i.e., amino terminal) end of the ABD-Fc molecule or can be at the 3' (i.e., carboxyl terminal) end of the ABD-Fc molecule. It is further provided that the bioactive agent may be associated between the ABD and the FcRn binding IgG Fc fragment, wherein either the ABD or the FcRn binding IgG Fc fragment is at the 5' (i.e., amino terminal) end of the bioactive agent. In a preferred embodiment, fusion proteins of the invention include a bioactive agent recombinantly fused or chemically conjugated to the ABD-Fc.

A bioactive agent can be any polypeptide or synthetic drug known to one of skill in the art. In one example, a bioactive agent is a polypeptide consisting of at least 5, preferably at least 10, at least 20, at least 30, at least 40, at least 50, at least 60, at least 70, at least 80, at least 90 or at least 100 amino acid residues. Examples of bioactive polypeptides include, but are not limited to, various types of antibodies, antigen binding domains (e.g., scFv, Fv, Fab, F(ab)₂, domain antibodies, and the like), cytokines (e.g., IL-1 (such as anakinra), IL-2, IL-3, IL-4, IL-5, IL-6, IL-7, IL-10, IL-12, IL-13, IL-15, IFN-gamma, IFN-alpha and IFN-beta), cell adhesion molecules (e.g., cadherins, such as cadherin-11, CTLA4, CD2, and CD28); ligands (e.g., TNFs such as TNF-alpha, TNF-beta); anti-angiogenic factors such as endostatin; receptors, antibodies and growth factors (e.g., PDGF and its receptor, EGF and its receptor, NGF and its receptor, and KGF, such as palifermin, and its receptor). Other examples of bioactive agents include recombinant erythropoietin such as Epoetin alfa, recombinant human granulocyte colony-stimulating factor (G-CSF) or filgrastim, alteplase, darbepoeitin alfa, domase alfa, entanercept, somatotropin, somatrem, or tenecteplase.

A bioactive agent can also be a therapeutic moiety such as a cytotoxin (e.g., a cytostatic or cytocidal agent), a therapeutic agent or a radioactive element (e.g., alpha-emitters, gamma-emitters, etc.). Examples of cytostatic or cytocidal agents include, but are not limited to, paclitaxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cisdichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC), and anti-mitotic agents (e.g., vincristine and vinblastine).

The present invention also encompasses fusing the ABD-Fc to a bioactive agent that is a diagnostic agent. The ABD-Fc-diagnostic molecule of the invention can be used diagnostically to, for example, monitor the development or progression of a disease, disorder or infection as part of a clinical testing procedure to, e.g., determine the efficacy of a given treatment regimen. Detection can be facilitated by coupling the pharmacologic enhancing molecule to a detectable substance. Examples of detectable substances include various enzymes, prosthetic groups, fluorescent materials, luminescent materials, bioluminescent materials, radioactive materials, positron emitting metals, and nonradioactive paramagnetic metal ions. The detectable substance may be coupled or conjugated either directly to the antibody or indirectly, through an intermediate (such as, for example, a linker known in the art) using techniques known in the art. See, for example, U.S. Pat. No. 4,741,900 for metal ions which can be conjugated to antibodies for use as diagnostics according to the present invention. Examples of suitable enzymes include horseradish peroxidase, alkaline phosphatase, O-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include 125I, 131I, 111In or 99mTc.

Techniques for conjugating the ABD-Fc molecule of the invention to a bioactive agent are well known; see, e.g., Arnon et al., "Monoclonal Antibodies For Immunotargeting Of Drugs In Cancer Therapy", in Monoclonal Antibodies And Cancer Therapy, Reisfeld et al. (eds.), 1985, pp. 243-56, Alan R. Liss, Inc.); Hellstrom et al., "Antibodies For Drug Delivery", in Controlled Drug Delivery (2nd Ed.), Robinson et al. (eds.), 1987, pp. 623-53, Marcel Dekker, Inc.); Thorpe, "Antibody Carriers Of Cytotoxic Agents In Cancer Therapy: A Review", in Monoclonal Antibodies '84:Biological And Clinical Applications, Pinchera et al. (eds.), 1985, pp. 475-506); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), 1985, pp. 303-16, Academic Press; and Thorpe et al., Immunol. Recombinant expression vector, 62:119-58, 1982.

### Methods of producing ABD-Fc-bioactive agents of the invention

ABD-Fc-bioactive agents of the invention can be produced by standard recombinant DNA techniques or by protein synthetic techniques, e.g., by use of a peptide synthesizer. For example, a nucleic acid molecule encoding an ABD-Fc-bioactive agent can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, e.g., Current Protocols in Molecular Biology, Ausubel et al., eds., John Wiley & Sons, 1992). Moreover, a nucleic acid encoding a bioactive agent can be cloned into an expression vector containing the ABD-Fc domain or a fragment thereof such that the bioactive agent is linked in-frame to the constant domain or fragment thereof.

Methods for fusing or conjugating polypeptides to the constant regions of antibodies are known in the art. See, e.g., U.S. Pat. Nos. 5,336,603, 5,622,929, 5,359,046, 5,349,053, 5,447,851, 5,723,125, 5,783,181, 5,908,626, 5,844,095, and 5,112,946; EP 307,434; EP 367,166; EP 394,827; PCT publications WO 91/06570, WO 96/04388, WO 96/22024, WO 97/34631, and WO 99/04813; Ashkenazi et al., Proc. Natl. Acad. Sci. USA, 88: 10535-10539, 1991; Traunecker et al., Nature, 331:84-86, 1988; Zheng et al., J. Immunol., 154:5590-5600, 1995; and Vil et al., Proc. Natl. Acad. Sci. USA, 89:11337-11341,1992.

The nucleotide sequence encoding a bioactive agent may be obtained from any information available to those of skill in the art (e.g., from Genbank, the literature, or by routine cloning), and the nucleotide sequence encoding a constant domain or a fragment thereof with increased affinity for the FcRn may be determined by sequence analysis of mutants produced using techniques described herein, or may be obtained from Genbank or the literature. The nucleotide sequence coding for an ABD-Fc-bioactive agent can be inserted into an appropriate expression vector, i.e., a vector which contains the necessary elements for the transcription and translation of the inserted protein-coding sequence. A variety of host-vector systems may be utilized in the present invention to express the protein-coding sequence. These include but are not limited to mammalian cell systems infected with virus (e.g., vaccinia virus, adenovirus, etc.); insect cell systems infected with virus (e.g., baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

The expression of An ABD-Fc-bioactive agent may be controlled by any promoter or enhancer element known in the art. Promoters which may be used to control the expression of the gene encoding fusion protein include, but are not limited to, the SV40 early promoter region (Bernoist and Chambon, Nature, 290:304-310, 1981), the promoter contained in the 3' long terminal repeat of Rous sarcoma virus (Yamamoto, et al., Cell, 22:787-797, 1980), the herpes thymidine kinase promoter (Wagner et al., Proc. Natl. Acad. Sci. U.S.A., 78:1441-1445, 1981), the regulatory sequences of the metallothionein gene (Brinster et al., Nature, 296:39-42, 1982), the tetracycline (Tet) promoter (Gossen et al., Proc. Nat. Acad. Sci. USA, 89:5547-5551, 1995); prokaryotic expression vectors such as the .beta.-lactamase promoter (Villa-Kamaroff, et al., Proc. Natl. Acad. Sci. U.S.A., 75:3727-3731, 1978), or the tac promoter (DeBoer, et al., Proc. Natl. Acad. Sci. U.S.A., 80:21-25, 1983; see also "Useful proteins from recombinant bacteria" in Scientific American, 242:74-94, 1980); plant expression vectors comprising the nopaline synthetase promoter region (Herrera-Estrella et al., Nature, 303:209-213, 1983) or the cauliflower mosaic virus .sup.35S RNA promoter (Gardner, et al., Nucl. Acids Res., 9:2871, 1981), and the promoter of the photosynthetic enzyme ribulose biphosphate carboxylase (Herrera-Estrella et al., Nature, 310:115-120, 1984); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., Cell 38:639-646, 1984; Ornitz et al., 50:399-409, Cold Spring Harbor Symp. Quant. Biol., 1986; MacDonald, Hepatology 7:425-515, 1987); insulin gene control region which is active in pancreatic beta cells (Hanahan, Nature 315:115-122, 1985), immunoglobulin gene control region which is active in lymphoid cells (Grosschedl et al., Cell, 38:647-658, 1984; Adames et al., Nature 318:533-538, 1985; Alexander et al., Mol. Cell. Biol., 7:1436-1444, 1987), mouse mammary tumor virus control region which is active in testicular, breast, lymphoid and mast cells (Leder et al., Cell, 45:485-495, 1986), albumin gene control region which is active in liver (Pinkert et al., Genes and Devel., 1:268-276, 1987), .alpha.-fetoprotein gene control region which is active in liver (Krumlauf et al., Mol. Cell. Biol., 5:1639-1648,1985; Hammer et al., Science, 235:53-58, 1987; a 1-antitrypsin gene control region which is active in the liver (Kelsey et al., Genes and Devel., 1:161-171, 1987), beta-globin gene control region which is active in myeloid cells (Mogram et al., Nature, 315:338-340, 1985; Kollias et al., Cell, 46:89-94, 1986; myelin basic protein gene control region which is active in oligodendrocyte cells in the brain (Readhead et al., Cell, 48:703-712, 1987); myosin light chain-2 gene control region which is active in skeletal muscle (Sani, Nature, 314:283-286, 1985); neuronal-specific enolase (NSE) which is active in neuronal cells (Morelli et al., Gen. Virol., 80:571-83, 1999); brain-derived neurotrophic factor (BDNF) gene control region which is active in neuronal cells (Tabuchi et al., Biochem. Biophysic. Res. Comprising., 253:818-823, 1998); glial fibrillary acidic protein (GFAP) promoter which is active in astrocytes (Gomes et al., Braz. J. Med. Biol. Res., 32(5):619-631, 1999; Morelli et al., Gen. Virol., 80:571-83, 1999) and gonadotropic releasing hormone gene control region which is active in the hypothalamus (Mason et al., Science, 234:1372-1378, 1986).

In a specific embodiment, the expression of an ABD-Fc-bioactive agent is regulated by a constitutive promoter. In another embodiment, the expression of a ABD-Fc-bioactive agent is regulated by an inducible promoter. In accordance with these embodiments, the promoter may be a tissue-specific promoter.

In mammalian host cells, a number of viral-based expression systems may be utilized. In cases where an adenovirus is used as an expression vector, the ABD-Fc-bioactive agent coding sequence may be ligated to an adenovirus transcription/translation control complex, e.g., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing the antibody molecule in infected hosts (e.g., see Logan & Shenk, Proc. Natl. Acad. Sci. USA, 81:355-359, 1984). Specific initiation signals may also be required for efficient translation of inserted ABD-Fc-bioactive agent coding sequences. These signals include the ATG initiation codon and adjacent sequences. Furthermore, the initiation codon must be in phase with the reading frame of the desired coding sequence to ensure translation of the entire insert. These exogeneous translation control signals and initiation codons can be of a variety of origins, both natural and synthetic. The efficiency of expression may be enhanced by the inclusion of appropriate transcription enhancer elements, transcription terminators, etc. (see Bitter et al., Methods in Enzymol., 153:516-544, 1987).

Expression vectors containing inserts of a gene encoding an ABD-Fc-bioactive agent can be identified by three general approaches: (a) nucleic acid hybridization, (b) presence or absence of "marker" gene functions, and (c) expression of inserted sequences. In the first approach, the presence of a gene encoding an ABD-Fc-bioactive agent in an expression vector can be detected by nucleic acid hybridization using probes comprising sequences that are homologous to an inserted gene encoding the fusion protein. In the second approach, the recombinant vector/host system can be identified and selected based upon the presence or absence of certain "marker" gene functions (e.g. thymidine kinase activity, resistance to antibiotics, transformation phenotype, occlusion body formation in baculovirus, etc.) caused by the insertion of a nucleotide sequence encoding an ABD-Fc-bioactive agent in the vector. For example, if the nucleotide sequence encoding the fusion protein is inserted within the marker gene sequence of the vector, recombinants containing the gene encoding the fusion protein insert can be identified by the absence of the marker gene function. In the third approach, recombinant expression vectors can be identified by assaying the gene product (i.e., fusion protein) expressed by the recombinant. Such assays can be based, for example, on the physical or functional properties of the fusion protein in in vitro assay systems, e.g., binding with anti-bioactive agent antibody.

In addition, a host cell strain may be chosen which modulates the expression of the inserted sequences, or modifies and processes the gene product in the specific fashion desired. Expression from certain promoters can be elevated in the presence of certain inducers; thus, expression of the genetically engineered fusion protein may be controlled. Furthermore, different host cells have characteristic and specific mechanisms for the translational and post-translational processing and modification (e.g., glycosylation, phosphorylation of proteins). Appropriate cell lines or host systems can be chosen to ensure the desired modification and processing of the foreign protein expressed. For example, expression in a bacterial system will produce an unglycosylated product and expression in yeast will produce a glycosylated product. Eukaryotic host cells which possess the cellular machinery for proper processing of the primary transcript, glycosylation, and phosphorylation of the gene product may be used. Such mammalian host cells include but are not limited to CHO, VERY, BHK, HeLa, COS, MDCK, 293, 3T3, W138, and in particular, neuronal cell lines such as, for example, SK-N-AS, SK-N-FJ, SK-N-DZ human neuroblastomas (Sugimoto et al., J. Natl. Cancer Inst., 73: 51-57, 1984), SK-N-SH human neuroblastoma (Biochim. Biophys. Acta, 704: 450-460, 1982), Daoy human cerebellar medulloblastoma (He et al., Cancer Res., 52: 1144-1148, 1992) DBTRG-05MG glioblastoma cells (Kruse et al., 1992, In Vitro Cell. Dev. Biol., 28A:609-614, 1992), IMR-32 human neuroblastoma (Cancer Res., 30: 2110-2118, 1970), 1321N1 human astrocytoma (Proc. Natl. Acad. Sci. USA, 74: 4816, 1997), MOG-G-CCM human astrocytoma (Br. J. Cancer, 49: 269, 1984), U87MG human glioblastoma-astrocytoma (Acta Pathol. Microbiol. Scand., 74: 465-486, 1968), A172 human glioblastoma (Olopade et al., Cancer Res., 52: 2523-2529 1992), C6 rat glioma cells (Benda et al., Science, 161: 370-371, 1968), Neuro-2a mouse neuroblastoma (Proc. Natl. Acad. Sci. USA, 65: 129-136, 1970), NB41A3 mouse neuroblastoma (Proc. Natl. Acad. Sci. USA, 48: 1184-1190, 1962), SCP sheep choroid plexus (Bolin et al., J. Virol. Methods, 48: 211-221, 1994), G355-5, PG-4 Cat normal astrocyte (Haapala et al., J. Virol., 53: 827-833, 1985), Mpf ferret brain (Trowbridge et al., In Vitro, 18: 952-960, 1982), and normal cell lines such as, for example, CTX TNA2 rat normal cortex brain (Radany et al., Proc. Natl. Acad. Sci. USA, 89: 6467-6471, 1992) such as, for example, CRL7030 and Hs578Bst. Furthermore, different vector/host expression systems may effect processing reactions to different degrees.

For long-term, high-yield production of recombinant proteins, stable expression is preferred. For example, cell lines which stably express the fusion protein may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched medium, and then are switched to a selective medium. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines that express the differentially expressed or pathway gene protein. Such engineered cell lines may be particularly useful in screening and evaluation of compounds that affect the endogenous activity of the differentially expressed or pathway gene protein.

A number of selection systems may be used, including but not limited to the herpes simplex virus thymidine kinase (Wigler, et al., Cell, 11:223, 1997), hypoxanthine-guanine phosphoribosyltransferase (Szybalska & Szybalski, Proc. Natl. Acad. Sci. USA, 48:2026, 1962), and adenine phosphoribosyltransferase (Lowy, et al., 1980, Cell, 22:817,1980) genes can be employed in tk-, hgprt- or aprt-cells, respectively. Also, antimetabolite resistance can be used as the basis of selection for dhfr, which confers resistance to methotrexate (Wigler, et al., Natl. Acad. Sci. USA, 77:3567,1980; O'Hare, et al., Proc. Natl. Acad. Sci. USA, 78:1527, 1981); gpt, which confers resistance to mycophenolic acid (Mulligan & Berg, Proc. Natl. Acad. Sci. USA, 78:2072, 1981); neo, which confers resistance to the aminoglycoside G-418 (Colberre-Garapin, et al., J. Mol. Biol., 150:1, 1981); and hygro, which confers resistance to hygromycin (Santerre, et al., Gene, 30:147, 1984) genes.

Once an ABD-Fc-bioactive agent of the invention has been produced by recombinant expression, it may be purified by any method known in the art for purification of a protein, for example, by chromatography (e.g., ion exchange, affinity, particularly by affinity for the specific antigen after Protein A, and sizing column chromatography), centrifugation, differential solubility, or by any other standard technique for the purification of proteins.

### Prophylactic and Therapeutic Uses of the ABD-Fc-bioactive agent

The present disclosure provides therapies which involve administering the ABD-Fc-bioactive agent to an animal, preferably a mammal, and most preferably a human, for preventing, treating, or ameliorating symptoms associated with a disease, disorder, or infection. In one example, the bioactive agent is an antibody, or an antigen binding fragment thereof. The ABD-Fc-bioactive agent may be provided in pharmaceutically acceptable compositions as known in the art or as described herein. The ABD-Fc-bioactive agent of the present invention can function as antagonists of a disease, disorder, or infection and can be administered to an animal, preferably a mammal and most preferably a human, to treat, prevent or ameliorate one or more symptoms associated with the disease, disorder, or infection. For example, the bioactive agent can be an antibody which can disrupt or prevent the interaction between a viral antigen and its host cell receptor and can be administered to an animal, preferably a mammal and most preferably a human, to treat, prevent or ameliorate one or more symptoms associated with a viral infection. Antibodies can also recognize and bind to target antigens found on the surface of diseased cells and tissues, such as, for example, cancerous cells and/or tumors or inflamed tissues and activate an immune response to treat, prevent or ameliorate the diseases caused by said cancerous cells and/or tumors or said inflamed tissues.

In one example, the ABD-Fc molecule of the invention further includes an antibody that can also be used to prevent, inhibit or reduce the growth or metastasis of cancerous cells. In a specific embodiment, an antibody inhibits or reduces the growth or metastasis of cancerous cells by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the growth or metastasis in absence of said antibody. In another embodiment, a combination of antibodies inhibits or reduces the growth or metastasis of cancer by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the growth or metastasis in absence of said antibodies. Examples of cancers include, but are not limited to, leukemia (e.g., acute leukemia such as acute lymphocytic leukemia and acute myelocytic leukemia), neoplasms, tumors (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder, carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma), heavy chain disease, metastases, or any disease or disorder characterized by uncontrolled cell growth.

In another example, the ABD-Fc molecule of the invention further includes an antibody that can can also be used to reduce the inflammation experienced by animals, particularly mammals, with inflammatory disorders. In a specific embodiment, an antibody reduces the inflammation in an animal by at least 99%, at least 95%, at least 90%, at least 85%, at least 80%, at least 75%, at least 70%, at least 60%, at least 50%, at least 45% at least 40%, at least 45%, at least 35%, at least 30%, at least 25%, at least 20%, or at least 10% relative to the inflammation in an animal in the not administered said antibody. In another embodiment, a combination of antibodies reduce the inflammation in an animal by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the inflammation in an animal in not administered said antibodies. Examples of inflammatory disorders include, but are not limited to, rheumatoid arthritis, spondyloarthropathies, inflammatory bowel disease, asthma, atopic allergy, chronic obstructive pulmonary disorder, cystic fibrosis, active systemic lupus erythematosus (SLE), lupus, sepsis and psoriasis. Examples of inflammatory symptoms include, but are not limited to, epithelial cell hyperplasia, excessive T cell, B cell, eosinophil, macrophage, monocyte, neutrophil, or mast cell activity, mucin overproduction, and bronchial hyperresponsiveness. In certain embodiments, the antibody used for treatment of inflammation (or cancer) is a modified anti-alphavbeta3 antibody, preferably a Vitaxin.RTM. antibody (see, PCT publications WO 98/33919 and WO 00/78815, both by Huse et al).

In yet another example, the ABD-Fc molecule of the invention further includes an antibody that can be used to prevent the rejection of transplants. Antibodies can also be used to prevent clot formation. Further, antibodies that function as agonists of the immune response can also be administered to an animal, preferably a mammal, and most preferably a human, to treat, prevent or ameliorate one or more symptoms associated with the disease, disorder, or infection.

In yet another example, the ABD-Fc molecule of the invention further includes antibodies that immunospecifically bind to one or more antigens and may be used locally or systemically in the body as a therapeutic. The ABD-Fc-polypeptides of this invention may also be advantageously utilized in combination with monoclonal or chimeric antibodies, or with lymphokines or hematopoietic growth factors (such as, e.g., IL-2, IL-3 and IL-7), which, for example, serve to increase the number or activity of effector cells which interact with the antibodies. The ABD-Fc molecules of the invention of this invention may also be advantageously utilized in combination with one or more drugs used to treat a disease, disorder, or infection such as, for example anti-cancer agents, anti-inflammatory agents or anti-viral agents. Examples of anti-cancer agents include, but are not limited to, isplatin, ifosfamide, paclitaxel, taxanes, topoisomerase I inhibitors (e.g. CPT-11, topotecan, 9-AC, and GG-211), gemcitabine, vinorelbine, oxaliplatin, 5-fluorouracil (5-FU), leucovorin, vinorelbine, temodal, and taxol. Examples of anti-viral agents include, but are not limited to, cytokines (e.g., IFN-.alpha., IFN-beta., IFN-.gamma.), inhibitors of reverse transcriptase (e.g., AZT, 3TC, D4T, ddC, ddI, d4T, 3TC, adefovir, efavirenz, delavirdine, nevirapine, abacavir, and other dideoxynucleosides or dideoxyfluoronucleosides), inhibitors of viral mRNA capping, such as ribavirin, inhibitors of proteases such HIV protease inhibitors (e.g., amprenavir, indinavir, nelfinavir, ritonavir, and saquinavir), amphotericin B, castanospermine as an inhibitor of glycoprotein processing, inhibitors of neuraminidase such as influenza virus neuraminidase inhibitors (e.g., zanamivir and oseltamivir), topoisomerase I inhibitors (e.g., camptothecins and analogs thereof), amantadine, and rimantadine. Examples of anti-inflammatory agents include, but are not limited to, nonsteroidal anti-inflammatory drugs such as COX-2 inhibitors (e.g., meloxicam, celecoxib, rofecoxib, flosulide, and SC-58635, and MK-966), ibuprofen and indomethacin, and steroids (e.g., deflazacort, dexamethasone and methylprednisolone).

In preferred embodiments, an ABD-Fc molecule of the invention extends in vivo half-lives of bioactive molecules such as those used in passive immunotherapy (for either therapy or prophylaxis). Because of the extended half-life, passive immunotherapy or prophylaxis can be accomplished using lower doses and/or less frequent administration of the therapeutic resulting in fewer side effects, better patient compliance, less costly therapy/prophylaxis, etc. In a preferred embodiment, the therapeutic/prophylactic is an ABD-Fc molecule of the invention further including an antibody that binds RSV, for example, SYNAGIS (palivizumab) or motavizumab (both from MedImmune, Inc., MD) or other anti-RSV antibodies. Such anti-RSV antibodies, and methods of administration are disclosed in U.S. Pat. Nos. 6,855,493 and 6,818,216, respectively, entitled "Methods of Administering/Dosing Anti-RSV Antibodies for Prophylaxis and Treatment," by Young et al.. Also included are the anti-RSV antibodies described in Section 5.1, supra.

### Administration of ABD-Fc-bioactive agents of the invention

The disclosure provides methods of treatment, prophylaxis, and amelioration of one or more symptoms associated with a disease, disorder or infection by administrating to a subject an effective amount of ABD-Fc-bioactive agent of the invention, or pharmaceutical composition comprising ABD-Fc-bioactive agent of the invention. The disclosure also provides methods of treatment, prophylaxis, and amelioration of one or more symptoms associated with a disease, disorder or infection by administering to a subject an effective amount of ABD-Fc-bioactive agent of the invention, or a pharmaceutical composition comprising a ABD-Fc-bioactive agent of the invention. In a preferred aspect, ABD-Fc-bioactive agent is substantially purified (i.e., substantially free from substances that limit its effect or produce undesired side-effects). In a specific embodiment, the subject is an animal, preferably a mammal such as non-primate (e.g., cows, pigs, horses, cats, dogs, rats etc.) and a primate (e.g., monkey such as a cynomolgous monkey and a human). In a preferred embodiment, the subject is a human.

Various delivery systems are known and can be used to administer an ABD-Fc-bioactive agent of the invention, e.g., encapsulation in liposomes, microparticles, microcapsules, recombinant cells capable of expressing the antibody or fusion protein, receptor-mediated endocytosis (see, e.g., Wu and Wu, J. Biol. Chem., 262:4429-4432, 1987), construction of a nucleic acid as part of a retroviral or other vector, etc. Methods of administering include, but are not limited to, parenteral administration (e.g., intradermal, intramuscular, intraperitoneal, intravenous and subcutaneous), epidural, and mucosal (e.g., intranasal, see e.g., WO 03/086443, and oral routes). In a specific embodiment, ABD-Fc-bioactive agent of the invention are administered intramuscularly, intravenously, or subcutaneously. The compositions may be administered by any convenient route, either systemic or local, for example by infusion or bolus injection, or by absorption through epithelial or mucocutaneous linings (e.g., oral mucosa, rectal and intestinal mucosa, etc.) and may be administered together with other biologically active agents. Administration can be systemic or local. In addition, pulmonary administration can also be employed, e.g., by use of an inhaler or nebulizer, and formulation with an aerosolizing agent. See, e.g., U.S. Pat. Nos. 6,019,968; 5,985,320; 5,985,309; 5,934,272; 5,874,064; 5,855,913; 5,290,540; and 4,880,078; and PCT Publication Nos. WO 92/19244; WO 97/32572; WO 97/44013; WO 98/31346; WO 99/66903, WO 04/058156, WO 03/087339, WO 03/087335 and WO 03/087327. Such pulmonary or intranasal or other mucosal administration may be employed to deliver the ABD-Fc-bioactive agent of the invention systemically. In a preferred embodiment, the ABD-Fc-bioactive agent of the invention is administered using Alkermes AIR pulmonary drug delivery technology (Alkermes, Inc., Cambridge, Mass.).

The invention also provides that the ABD-Fc-bioactive agent of the invention is packaged in a hermetically sealed container such as an ampoule or sachette indicating the quantity of the ABD-Fc-bioactive agent of the invention. In one embodiment, the ABD-Fc-bioactive agent molecule of the invention is supplied as a dry sterilized lyophilized powder or water free concentrate in a hermetically sealed container and can be reconstituted, e.g., with water or saline to the appropriate concentration for administration to a subject. Preferably, ABD-Fc-bioactive agent molecule of the invention is supplied as a dry sterile lyophilized powder in a hermetically sealed container at a unit dosage of at least 5 mg, more preferably at least 10 mg, at least 15 mg, at least 25 mg, at least 35 mg, at least 45 mg, at least 50 mg, or at least 75 mg. The lyophilized ABD-Fc-bioactive agent molecule of the invention should be stored at between 2 and 8°C in its original container and the ABD-Fc-bioactive agent molecule of the invention should be administered within 12 hours, preferably within 6 hours, within 5 hours, within 3 hours, or within 1 hour after being reconstituted. In an alternative embodiment, the ABD-Fc-bioactive agent molecule of the invention is supplied in liquid form in a hermetically sealed container indicating the quantity and concentration of the ABD-Fc-bioactive agent molecule of the invention. Preferably, the ABD-Fc-bioactive agent molecule of the invention is supplied in a hermetically sealed container at least 1 mg/ml, more preferably at least 2.5 mg/ml, at least 5 mg/ml, at least 8 mg/ml, at least 10 mg/ml, at least 15 mg/kg, or at least 25 mg/ml.

In a specific embodiment, it may be desirable to administer the pharmaceutical compositions of the invention locally to the area in need of treatment; this may be achieved by, for example, and not by way of limitation, local infusion, by injection, or by means of an implant, said implant being of a porous, non-porous, or gelatinous material, including membranes, such as silastic membranes, or fibers. Preferably, when administering an ABD-Fc-bioactive agent of the invention, care must be taken to use materials to which the ABD-Fc-bioactive agent of the invention does not absorb.

In another embodiment, the composition can be delivered in a vesicle, in particular a liposome (see Langer, Science, 249:1527-1533, 1990; Treat et al., in Liposomes in the Therapy of Infectious Disease and Cancer, Lopez-Berestein and Fidler (eds.), Liss, New York, pp. 353-365 (1989); Lopez-Berestein, ibid., pp. 3 17-327; see generally ibid.).

In yet another embodiment, the composition can be delivered in a confrolled release or sustained release system. Any technique known to one of skill in the art can be used to produce sustained release formulations comprising one or more antibodies, or one or more fusion proteins. See, e.g. U.S. Pat. No. 4,526,938; PCT publication WO 91/05548; PCT publication WO 96/20698; Ning et al., "Intratumoral Radioimmunotheraphy of a Human Colon Cancer Xenograft Using a Sustained-Release Gel," Radiotherapy & Oncology, 39:179-189, 1996; Song et al., "Antibody Mediated Lung Targeting of Long-Circulating Emulsions," PDA Journal of Pharmaceutical Science & Technology, 50:372-397,1995; Cleek et al., "Biodegradable Polymeric Carriers for a bFGF Antibody for Cardiovascular Application," Pro. Intl. Symp. Control. Rel. Bioact. Mater., 24:853-854, 1997; and Lam et al., "Microencapsulation of Recombinant Humanized Monoclonal Antibody for Local Delivery," Proc. Int'l. Symp. Control Rel. Bioact. Mater., 24:759-760, 1997. In one embodiment, a pump may be used in a controlled release system (see Langer, supra; Sefton, CRC Crit. Ref. Biomed. Eng., 14:20, 1987; Buchwald et al., Surgery, 88:507, 1980; and Saudek et al., N. Engl. J. Med., 321:574, 1989). In another embodiment, polymeric materials can be used to achieve controlled release of antibodies or fusion proteins (see e.g., Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, N.Y. (1984); Ranger and Peppas, J., Macromol. Sci. Rev. Macromol. Chem., 23:61,1983; see also Levy et al., Science, 228:190, 1985; During et al., Ann. Neurol., 25:351, 1989; Howard et al., J. Neurosurg., 7 1:105, 1989); U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253). In yet another embodiment, a controlled release system can be placed in proximity of the therapeutic target (e.g., the lungs), thus requiring only a fraction of the systemic dose (see, e.g., Goodson, in Medical Applications of Controlled Release, supra, vol. 2, pp. 115-138 (1984)).

Other controlled release systems are discussed in the review by Langer, Science, 249:1527-1533,1990).

In a specific embodiment where the composition of the invention is a nucleic acid encoding an ABD-Fc molecule of the invention including a bioactive agent, the nucleic acid can be administered in vivo to promote expression of its encoded ABD-Fc-bioactive agent molecule of the invention, by constructing it as part of an appropriate nucleic acid expression vector and administering it so that it becomes intracellular, e.g., by use of a retroviral vector (see U.S. Pat. No. 4,980,286), or by direct injection, or by use of microparticle bombardment (e.g., a gene gun; Biolistic, Dupont), or coating with lipids or cell-surface receptors or transfecting agents, or by administering it in linkage to a homeobox-like peptide which is known to enter the nucleus (see e.g., Joliot et al., Proc. Natl. Acad. Sci. USA, 88:1864-1868, 1991), etc. Alternatively, a nucleic acid can be introduced intracellularly and incorporated within host cell DNA for expression by homologous recombination.

The present invention also provides pharmaceutical compositions. Such compositions comprise a prophylactically or therapeutically effective amount of an ABD-Fc-bioactive agent of the invention and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant (e.g., Freund's complete and incomplete, mineral gels such as aluminum hydroxide, surface active substances such as lysolecithin, pluronic polyols, polyanions, peptides, oil emulsions, keyhole limpet hemocyanins, dinitrophenol, and potentially useful adjuvants for humans such as BCG (Bacille Calmette-Guerin) and Corynebacterium parvum), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a prophylactically or therapeutically effective amount of the ABD-Fc molecule of the invention that further includes a bioactive agent, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection.

Generally, the ingredients of compositions of the invention are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration. Where the composition is administered by a pulmonary (i.e., inhalation) or intranasal route, either a lyophilized powder to be subsequently reconstituted, or a stable liquid formulation can be filled into vials or a syringe or an atomizer.

The compositions of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The amount of the composition of the invention which will be effective in the treatment, prevention or amelioration of one or more symptoms associated with a disease, disorder, or infection can be determined by standard clinical techniques. The precise dose to be employed in the formulation will depend on the route of administration, the age of the subject, and the seriousness of the disease, disorder, or infection, and should be decided according to the judgment of the practitioner and each patient's circumstances. Effective doses may be extrapolated from dose-response curves derived from in vitro or animal model (e.g., the cotton rat or Cynomolgous monkey) test systems.

For fusion proteins which include the ABD-Fc-bioactive agent of the invention, the therapeutically or prophylactically effective dosage administered to a subject ranges from about 0.001 to 50 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight or 0.3 to 3 mg/kg body weight or 3 to 15 mg/kg body weight. For antibodies, the therapeutically or prophylactically effective dosage administered to a subject is typically 0.1 mg/kg to 200 mg/kg of the subject's body weight. Preferably, the dosage administered to a subject is between 0.1 mg/kg and 20 mg/kg of the subject's body weight or 0.3 to 3 mg/kg body weight or 3 to 15 mg/kg body weight and more preferably the dosage administered to a subject is between 1 mg/kg to 10 mg/kg of the subject's body weight. The dosage will, however, depend upon the extent to which the in vivo half-life of the molecule has been increased. Generally, human antibodies and human fusion proteins have longer half-lives within the human body than antibodies of fusion proteins from other species due to the immune response to the foreign polypeptides. Thus, lower dosages of human antibodies or human fusion proteins and less frequent administration is often possible. Further, the dosage and frequency of administration of antibodies, fusion proteins, or conjugated molecules may be reduced also by enhancing uptake and tissue penetration (e.g., into the lung) of the antibodies or fusion proteins by modifications such as, for example, lipidation.

Treatment of a subject with a therapeutically or prophylactically effective amount of an ABD-Fc-bioactive agent of the invention can include a single treatment or, preferably, can include a series of treatments. In a preferred example, a subject is treated with the fusion protein in the range of between about 0.1 to 30 mg/kg body weight, one time per week for between about 1 to 10 weeks, preferably between 2 to 8 weeks, more preferably between about 3 to 7 weeks, and even more preferably for about 4, 5, or 6 weeks. In other embodiments, the pharmaceutical composition of the invention is administered once a day, twice a day, or three times a day. In other embodiments, the pharmaceutical composition is administered once a week, twice a week, once every two weeks, once a month, once every six weeks, once every two months, twice a year or once per year. It will also be appreciated that the effective dosage of the fusion protein used for treatment, whether therapeutically or prophylactically may increase or decrease over the course of a particular treatment.

### Gene Therapy

In a specific embodiment, nucleic acids comprising sequences encoding the ABD-Fc-bioactive agent of the invention, are administered to treat, prevent or ameliorate one or more symptoms associated with a disease, disorder, or infection, by way of gene therapy. Gene therapy refers to therapy performed by the administration to a subject of an expressed or expressible nucleic acid. In this embodiment of the disclosure, the nucleic acids produce their encoded ABD-Fc-bioactive agent that mediates a therapeutic or prophylactic effect.

Any of the methods for gene therapy available in the art can be used according to the present invention. Exemplary methods are described below.

For general reviews of the methods of gene therapy, see Goldspiel et al., Clinical Pharmacy, 12:488-505, 1993; Wu and Wu, Biotherapy, 3:87-95, 1991; Tolstoshev, Ann. Rev. Pharmacol. Toxicol., 32:573-596, 1993; Mulligan, Science, 260:926-932, 1993; and Morgan and Anderson, Ann. Rev. biochem. 62:191-217,1993; TIBTECH 11(5):155-215, 1993. Methods commonly known in the art of recombinant DNA technology which can be used are described in Ausubel et al. (eds.), Current Protocols in Molecular Biology, John Wiley & Sons, NY (1993); and Kriegler, Gene Transfer and Expression, A Laboratory Manual, Stockton Press, NY (1990).

In a preferred aspect, a composition of the invention comprises nucleic acids encoding an ABD-Fc agent together with a bioactive agent such as a therapeutic antibody, said nucleic acids being part of an expression vector that expresses the antibody in a suitable host. In particular, such nucleic acids have promoters, preferably heterologous promoters, operably linked to the antibody coding region, said promoter being inducible or constitutive, and, optionally, tissue-specific. In another particular embodiment, nucleic acid molecules are used in which the ABD-Fc-Antibody coding sequences and any other desired sequences are flanked by regions that promote homologous recombination at a desired site in the genome, thus providing for intrachromosomal expression of the antibody encoding nucleic acids (Koller and Smithies, Proc. Natl. Acad. Sci. USA, 86:8932-8935, 1989; and Zijlstra et al., Nature, 342:435-438, 1989).

In another preferred aspect, a composition of the invention comprises nucleic acids encoding a ABD-Fc-bioactive agent, said nucleic acids being a part of an expression vector that expression the ABD-Fc-bioactive agent in a suitable host. In particular, such nucleic acids have promoters, preferably heterologous promoters, operably linked to the coding region of a ABD-Fc-bioactive agent, said promoter being inducible or constitutive, and optionally, tissue-specific.

Delivery of the nucleic acids into a subject may be either direct, in which case the subject is directly exposed to the nucleic acid or nucleic acid-carrying vectors, or indirect, in which case, cells are first transformed with the nucleic acids in vitro, then transplanted into the subject. These two approaches are known, respectively, as in vivo or ex vivo gene therapy.

In a specific embodiment, the nucleic acid sequences are directly administered in vivo, where it is expressed to produce the encoded product. This can be accomplished by any of numerous methods known in the art, e.g., by constructing them as part of an appropriate nucleic acid expression vector and administering it so that they become intracellular, e.g., by infection using defective or attenuated retroviral or other viral vectors (see U.S. Pat. No. 4,980,286), or by direct injection of naked DNA, or by use of microparticle bombardment (e.g., a gene gun; Biolistic, Dupont), or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, e.g., Wu and Wu, J. Biol. Chem., 262:4429-4432, 1987) (which can be used to target cell types specifically expressing the receptors), etc. In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted in vivo for cell specific uptake and expression, by targeting a specific receptor (see, e.g., PCT Publications WO 92/06180; WO 92/22635; WO 92/20316; WO 93/14188; WO 93/20221). Alternatively, the nucleic acid can be introduced intracellularly and incorporated within host cell DNA for expression, by homologous recombination (Koller and Smithies, Proc. Natl. Acad. Sci. USA, 86:8932-8935, 1989; and Zijlstra et al., Nature, 342:435-438, 1989).

In a specific embodiment, viral vectors that contain nucleic acid sequences encoding ABD-Fc-bioactive agent are used. For example, a retroviral vector can be used (see Miller et al:, Meth. Enzymol., 217:581-599, 1993). These retroviral vectors contain the components necessary for the correct packaging of the viral genome and integration into the host cell DNA. The nucleic acid sequences encoding ABD-Fc-bioactive agent including a bioactive agent to be used in gene therapy are cloned into one or more vectors, which facilitates delivery of the nucleotide sequence into a subject. More detail about retroviral vectors can be found in Boesen et al., Biotherapy, 6:291-302, 1994, which describes the use of a retroviral vector to deliver the mdr 1 gene to hematopoietic stem cells in order to make the stem cells more resistant to chemotherapy. Other references illustrating the use of retroviral vectors in gene therapy are: Clowes et al., J. Clin. Invest., 93:644-651, 1994; Klein et al., Blood 83:1467-1473, 1994; Salmons and Gunzberg, Human Gene Therapy, 4:129-141, 1993; and Grossman and Wilson, Curr. Opin. in Genetics and Devel., 3:110-114, 1993.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, Current Opinion in Genetics and Development, 3:499-503, 1993, present a review of adenovirus-based gene therapy. Bout et al., Human Gene Therapy, 5:3-10, 1994, demonstrated the use of adenovirus vectors to transfer genes to the respiratory epithelia of rhesus monkeys. Other instances of the use of adenoviruses in gene therapy can be found in Rosenfeld et al., Science, 252:431-434, 1991; Rosenfeld et al., Cell, 68:143-155, 1992; Mastrangeli et al., J. Clin. Invest., 91:225-234,1993; PCT Publication WO 94/12649; and Wang et al., Gene Therapy, 2:775-783, 1995. In a preferred embodiment, adenovirus vectors are used.

Adeno-associated virus (AAV) has also been proposed for use in gene therapy (see, e.g., Walsh et al., Proc. Soc. Exp. Biol. Med., 204:289-300,1993, and U.S. Pat. No. 5,436,146).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a subject.

In this embodiment, the nucleic acid is introduced into a cell prior to administration in vivo of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcellmediated gene transfer, spheroplast fusion, etc. Numerous techniques are known in the art for the introduction of foreign genes into cells (see, e.g., Loeffler and Behr, Meth. Enzymol., 217:599-618, 1993; Cohen et al., Meth. Enzymol., 217:618-644, 1993; and Clin. Pharma. Ther., 29:69-92, 1985) and may be used in accordance with the present disclosure, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a subject by various methods known in the art. Recombinant blood cells (e.g., hematopoietic stem or progenitor cells) are preferably administered intravenously. The amount of cells envisioned for use depends on the desired effect, patient state, etc., and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T lymphocytes, B lymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood, fetal liver, etc.

In a preferred embodiment, the cell used for gene therapy is autologous to the subject.

In an embodiment in which recombinant cells are used in gene therapy, nucleic acid sequences encoding ABD-Fc-bioactive agent are introduced into the cells such that they are expressible by the cells or their progeny, and the recombinant cells are then administered in vivo for therapeutic effect. In a specific embodiment, stem or progenitor cells are used. Any stem and/or progenitor cells which can be isolated and maintained in vitro can potentially be used in accordance with this embodiment of the present invention (see e.g., PCT Publication WO 94/08598; Stemple and Anderson, Cell, 7 1:973-985, 1992; Rheinwald, Meth. Cell Bio., 21A:229, 1980; and Pittelkow and Scott, Mayo Clinic Proc., 61:771, 1986).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

### Characterization and Demonstration of Therapeutic or Prophylactic Utility

ABD-Fc-bioactive agent of the present invention may be characterized in a variety of ways. In particular, the activity of the bioactive agent can be characterized. In one embodiment, the ABD-Fc includes a bioactive agent which is an antibody or fragment thereof. The activity of the antibody, or fragment, can be assayed for its ability to immunospecifically bind to an antigen. Such an assay may be performed in solution (e.g., Houghten, Bio/Techniques, 13:412-421, 1992), on beads (Lam, Nature, 354:82-84, 1991, on chips (Fodor, Nature, 364:555-556,1993), on bacteria (U.S. Pat. No. 5,223,409), on spores (U.S. Pat. Nos. 5,571,698; 5,403,484; and 5,223,409), on plasmids (Cull et al., Proc. Natl. Acad. Sci. USA, 89:1865-1869, 1992) or on phage (Scott and Smith, Science, 249:386-390,1990; Devlin, Science, 249:404-406, 1990; Cwirla et al., Proc. Natl. Acad. Sci. USA, 87:6378-6382, 1990; and Felici, J. Mol. Biol., 222:301-310, 1991). Antibodies that have been identified to immunospecifically bind to an antigen or a fragment thereof can then be assayed for their specificity affinity for the antigen.

The binding affinity of an antibody of an ABD-Fc molecule to an antigen and the off-rate of an antibody-antigen interaction can be determined by competitive binding assays. One example of a competitive binding assay is a radioimmunoassay comprising the incubation of labeled antigen (e.g., 3H or 125I) with the antibody of interest in the presence of increasing amounts of unlabeled antigen, and the detection of the antibody bound to the labeled antigen. The affinity of the antibody of the present invention or a fragment thereof for the antigen and the binding off-rates can be determined from the saturation data by scatchard analysis. Competition with a second antibody can also be determined using radioimmunoassays. In this case, the antigen is incubated with an antibody of the present invention or a fragment thereof conjugated to a labeled compound (e.g., 3H or 125I) in the presence of increasing amounts of an unlabeled second antibody.

In a preferred embodiment, BIAcore kinetic analysis is used to determine the binding on and off rates of antibodies to an antigen. BIAcore kinetic analysis comprises analyzing the binding and dissociation of an antigen from chips with immobilized antibodies on their surface (see the Example section infra).

The ABD-Fc including a bioactive agent such as an antibody can also be assayed for their ability to inhibit the binding of an antigen to its host cell receptor using techniques known to those of skill in the art. For example, cells expressing the receptor for a viral antigen can'be contacted with virus in the presence or absence of an antibody and the ability of the antibody to inhibit viral antigen's binding can measured by, for example, flow cytometry or a scintillation counter. The antigen or the antibody can be labeled with a detectable compound such as a radioactive label (e.g. 32P, 35S, and 125I) or a fluorescent label (e.g., fluorescein isothiocyanate, rhodamine, phycoerythrin, phycocyanin, allophycocyanin, o-phthaldehyde and fluorescamine) to enable detection of an interaction between the antigen and its host cell receptor. Alternatively, the ability of antibodies to inhibit an antigen from binding to its receptor can be determined in cell-free assays. For example, virus or a viral antigen (e.g., RSV F glycoprotein) can be contacted in a cell-free assay with an antibody and the ability of the antibody to inhibit the virus or the viral antigen from binding to its host cell receptor can be determined. Preferably, the antibody is immobilized on a solid support and the antigen is labeled with a detectable compound. Alternatively, the antigen is immobilized on a solid support and the antibody is labeled with a detectable compound. The antigen may be partially or completely purified (e.g., partially or completely free of other polypeptides) or part of a cell lysate. Further, the antigen may be an ABD-Fc-bioactive agent comprising the viral antigen and a domain such as glutathionine-5-transferase. Alternatively, an antigen can be biotinylated using techniques well known to those of skill in the art (e.g., biotinylation kit, Pierce Chemicals; Rockford, Ill.).

The ABD-Fc-bioactive agent and compositions of the invention are preferably tested in vitro, and then in vivo for the desired therapeutic or prophylactic activity, prior to use in humans. For example, in vitro assays which can be used to determine whether administration of a specific ABD-Fc-bioactive agent or a composition of the present invention is indicated, include in vitro cell culture assays in which a subject tissue sample is grown in culture, and exposed to or otherwise administered ABD-Fc-bioactive agent, or composition of the present invention, and the effect of such ABD-Fc-bioactive agent, or a composition of the present invention upon the tissue sample is observed. In various specific embodiments, in vitro assays can be carried out with representative cells of cell types involved in a disease or disorder, to determine if ABD-Fc-bioactive agent, or composition of the present invention has a desired effect upon such cell types. Preferably, the ABD-Fc-bioactive agent, or compositions of the invention are also tested in in vitro assays and animal model systems prior to administration to humans.

ABD-Fc-bioactive agent, or compositions of the present invention for use in therapy can be tested for their toxicity in suitable animal model systems, including but not limited to rats, mice, cows, monkeys, and rabbits. For in vivo testing for the toxicity of ABD-Fc-bioactive agent, or a composition, any animal model system known in the art may be used.

Efficacy in treating or preventing viral infection may be demonstrated by detecting the ability of an ABD-Fc-bioactive agent, or a composition of the invention to inhibit the replication of the virus, to inhibit transmission or prevent the virus from establishing itself in its host, or to prevent, ameliorate or alleviate one or more symptoms associated with viral infection. The treatment is considered therapeutic if there is, for example, a reduction is viral load, amelioration of one or more symptoms or a decrease in mortality and/or morbidity following administration of ABD-Fc-bioactive agent, or a composition of the invention. ABD-Fc-bioactive agent, or compositions of the invention can also be tested for their ability to inhibit viral replication or reduce viral load in in vitro and in vivo assays.

Efficacy in treating or preventing bacterial infection may be demonstrated by detecting the ability of ABD-Fc-bioactive agent or a composition of the invention to inhibit the bacterial replication, or to prevent, ameliorate or alleviate one or more symptoms associated with bacterial infection. The treatment is considered therapeutic if there is, for example, a reduction is bacterial numbers, amelioration of one or more symptoms or a decrease in mortality and/or morbidity following administration of ABD-Fc-bioactive agent or a composition of the invention.

Efficacy in treating cancer may be demonstrated by detecting the ability ofABD-Fc-bioactive agent, or a composition of the invention to inhibit or reduce the growth or metastasis of cancerous cells or to ameliorate or alleviate one or more symptoms associated with cancer. The treatment is considered therapeutic if there is, for example, a reduction in the growth or metastasis of cancerous cells, amelioration of one or more symptoms associated with cancer, or a decrease in mortality and/or morbidity following administration of ABD-Fc-bioactive agent, or a composition of the invention. ABD-Fc-bioactive agent or compositions of the invention can be tested for their ability to reduce tumor formation in in vitro, ex vivo, and in vivo assays.

Efficacy in treating inflammatory disorders may be demonstrated by detecting the ability of an ABD-Fc-bioactive agent, or a composition of the invention to reduce or inhibit the inflammation in an animal or to ameliorate or alleviate one or more symptoms associated with an inflammatory disorder. The treatment is considered therapeutic if there is, for example, a reduction is in inflammation or amelioration of one or more symptoms following administration of ABD-Fc-bioactive agent, or a composition of the invention.

### Diagnostic Uses

ABD-Fc-bioactive agent of the invention can be used for diagnostic purposes to detect, diagnose, or monitor diseases, disorders or infections. The invention provides for the detection or diagnosis of a disease, disorder or infection, comprising: (a) assaying the expression of an antigen in cells or a tissue sample of a subject using one or more antibodies that immunospecifically bind to the antigen; and (b) comparing the level of the antigen with a control level, e.g., levels in normal tissue samples, whereby an increase in the assayed level of antigen compared to the control level of the antigen is indicative of the disease, disorder or infection. The disclosure also provides for the detection or diagnosis of a disease, disorder or infection, comprising (a) assaying the expression of an antigen in cells or a tissue sample of a subject using ABD-Fc including a bioactive agent of the invention that bind to the antigen; and (b) comparing the level of the antigen with a control level, e.g., levels in normal tissue samples, whereby an increase of antigen compared to the control level of the antigen is indicative of the disease, disorder or infection. Accordingly, the ABD-Fc includes a bioactive agent such as a ligand, cytokine or growth factor and the hinge-Fc region or fragments thereof, wherein the ABD-Fc includes a bioactive agent is capable of binding to an antigen being detected.

ABD-Fc including a bioactive agent of the invention can be used to assay antigen levels in a biological sample using, for example, SDS-PAGE and immunoassays known to those of skill in the art.

One aspect of the disclosure is the detection and diagnosis of a disease, disorder, or infection in a human. In one embodiment, diagnosis comprises: a) administering (for example, parenterally, subcutaneously, or intraperitoneally) to a subject an effective amount of a labeled ABD-Fc including a bioactive agent such asa an antibody that immunospecifically binds to an antigen; b) waiting for a time interval following the administration for permitting the labeled antibody to preferentially concentrate at sites in the subject where the antigen is expressed (and for unbound labeled molecule to be cleared to background level); c) determining background level; and d) detecting the labeled antibody in the subject, such that detection of labeled antibody above the background level indicates that the subject has the disease, disorder, or infection. In accordance with this embodiment, the antibody is labeled with an imaging moiety which is detectable using an imaging system known to one of skill in the art. Background level can be determined by various methods including, comparing the amount of labeled molecule detected to a standard value previously determined for a particular system.

### EXAMPLES

The following examples, including the experiments conducted and results achieved are provided for illustrative purposes only and are not to be construed as limiting upon the teachings herein.

### Example 1: Generating ABD-Fc Fusion Constructs:

An open reading frame encoding 42 amino acids of Albumin Binding Domain "ABD" (Johannson et al., 2002, supra) protein was constructed using several sequential polymerase chain reactions to assemble 8 overalapping nucleotides. The ABD ORF was inserted N terminal to the constant region (Fc) of murine Igg2A into a mammalian expression vector via NheI and Kas I sites. The final parental construct ABD-Fc contained 46 amino acids of ABD followed by the residues 216 through 460 of murine Igg 2A Fc which include the hinge region, CH2 and CH3 domains respectively. Following C terminal CH3 domain, a tandem c-Myc and His tags separated by a flexible (Gly4)Ser linker were inserted for subsequent pharmacokinetic studies. The integrity of the fusion construct was confirmed by DNA sequencing. All of the mutants of the ABD-Fc parental construct were made using overlap PCR strategy and inserted into a mammalian expression vector using either KasI/NheI or NheI/NotI restriction sites. The following nomenclature was used to describe the ABD-Fc mutants:
ABD-Fc: fusion of unmodified ABD and unmodified Fc (binding to both HSA/MSA and FcRn)
ABD-TM-Fc: triple knockout mutations of ABD residues S18A; T20A and Y21A (reduced binding of ABD to HSA/MSA);
ABD-Fc (His 310): knockout substitution in Fc region of Igg2A H310A (reduced binding of Fc to FcRn); and
ABD-Fc-DM: a combined triple mutation in ABD (S18A; Y20A and Y21A) and Fc (H310A). Reduced binding of both ABD to HSA/MSA and Fc to FcRn.

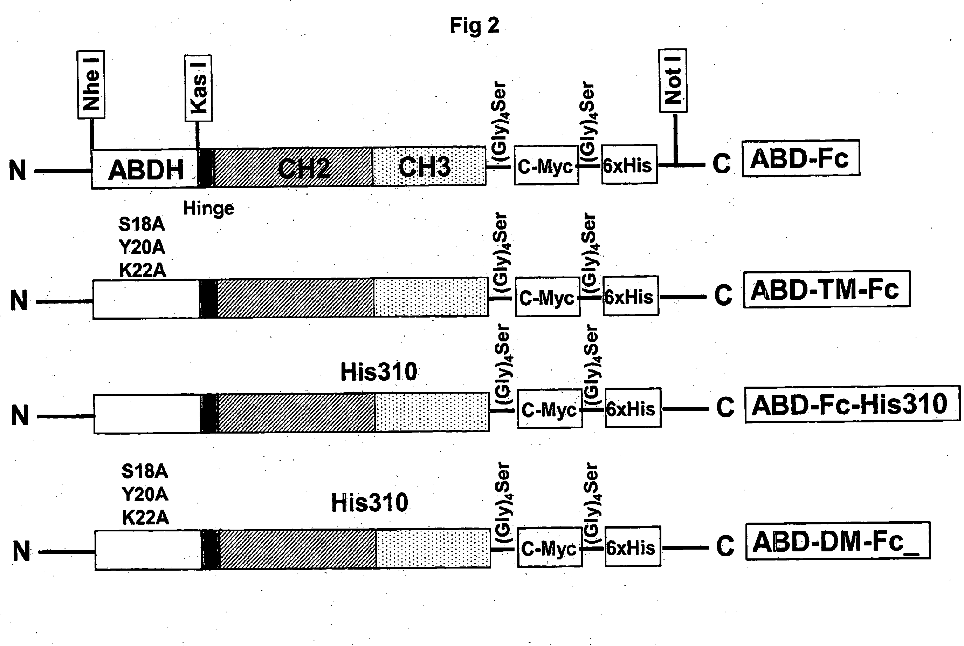

See Figure 2 for a schematic of the different variants. Sequences are shown below:

The final constructs were transiently transfected into Human Embryonic Kidney (HEK 293 cells) using Lipofectomine (Invitrogen, Inc, Carlsbad CA) and standard protocols. ABD-Fc fusion proteins were typically harvested 72 and 144 hours post transfection and purified from the conditioned media directly on the HiTrap Protein A column according to manufacturer's instructions (GE Healthcare). ABD-Fc (His 310) and ABD-Fc-DM could not be purified by HiTrapA column due to impaired protein A binding sites. Therefore, the conditioned media containing these proteins was first buffer exchanged into 50mM Hepes pH7.5; 150mM NaCl; 10mM Immidazole and purified by HiTrap NiNTA column according to manufacturer's instructions (GE Healthcare). As a last step of purification, all proteins were passed through S-200 size exclusion column (GE Healthcare). Protein purity was over 98% as accessed by SDS PAGE under reducing and non-reducing conditions.

### Example 2: Testing ABD-Fc for binding to HSA using biacore measurements

The interaction between different ABD-Fc variants and MSA (mouse serum albumin), HSA (human serum albumin) and mFcRn were monitored by surface plasmon resonance detection using BIAcore 3000 instrument. (GE Healthcare). First, ABD-Fc variants were coupled to dextran matrix of CM5 sensor chip using an Amine Coupling Kit at a surface density of 1000-2000 RU according to manufacturer's instructions. Human and mouse albumin as well mouse FcRn were flown over in a test-bind experiments at the concentrations ranging from 0.1-10µM at a flow rate of 5µl/min. Dilution and binding experiments were carried out in PBS pH7.5; 150mM NaCl; 0.005% Tween for albumin binding and 50mM NaPhosphate pH6.0; 150mM NaCl; 0.005% Tween for mouse FcRn. Subsequently, the format of the Biacore experiments were flipped where mouse and human albumin as well as mouse FcRn were coupled to a CM5 chip using Amine Coupling Kit at surface densities ranging from 500-1500 RU. The ABD-Fc variants were flown over at three different concentrations 0.5; 1 and 10µM. The latter format was more successful due to higher signal and self-consistency.

Although equilibrium binding constants (Kd) were not determined, three concentrations of the injectant (ABD-Fc or mouse FcRn depending on the BIAcore format (0.1µM;1µM and 10µM) were tested to ascertain that binding is specific. As predicted ABD-Fc binds to HSA, MSA and mouse FcRn. When a triple mutation is introduced into ABD, ABD-TM-Fc, it can no longer bind albumin but retains binding to mouse FcRn. The ABD-Fc variant with the mutation in the Fc region (ABD-Fc (His 310) can no longer bind to FcRn but retains ability to bind albumin. The double mutant (ABD-Fc-DM) with albumin and FcRn sites mutated doesn't interact with either protein under these experimental conditions.

### Example 3: Multi-Angle Static Light Scattering

Static light scattering measurements were performed at 25°C using a three-angle light scattering detector (Wyatt Technologies, Santa Barbara, CA) and a differential refractive index detector (Wyatt Optilab DSP; Wyatt Technologies) running in-line with an HPLC system. Samples were dialyzed overnight at 4°C against running buffer (150 mM NaCl, 50mM NaPhosphate ph 6.0 and 0.01% NaN₃). Samples were injected onto a G3000PWXL (TosoHaas, Montgomeryville, PA) size exclusion column equilibrated with running buffer at loading concentrations between 1.0 and 3.5 mg/ml (approximately 80 to 200µM protein). Data acquisition and analysis were performed using ASTRA 5.0 software (Wyatt Technologies).

To determine the stoichiometry of the ABD-Fc/albumin, ABD-Fc/mFcRn and ABD-Fc/albumin/mFcRn interaction, we have used multi-angle static light scattering (MASLS). This technique when coupled with size exclusion chromatography provides the weight-averaged molecular weight at each point of the chromatographic elution profile (Wyatt, 1993). At 4:1 molar ratio, mFcRn and ABD-Fc elute together at the molecular weight of 164kD and 259kD corresponding to 1:1 and 2:1 complex, respectively. This result is consistent with the notion that FcRn can bind to Fc region of the antibody with 2:1 stoichiometry. This data also suggests that fusion of the ABD polypeptide to the Fc does not interfere with the formation of the 2:1 complex albeit 1:1 complex is also present. At 2:1 it was observed that the molar ratio of human serum albumin to ABD exhibited a weaker 1:1 complex (with apparent molecular weight of ∼145kD) that partly dissociates on the gel filtration column. This result confirms the interaction between albumin and ABD in solution while the rapid dissociation on the gel filtration column suggests that the affinity of ABD-Fc to HSA is weaker than that to mouse FcRn. When the formation of the trimolecular complex (ABD-Fc/HSA/mFcRn) was tested using MASLS, a shift in the molecular weight was observed corresponding to the formation of the higher molecular weight species. However, we were not able to definitively determine respective solution stoichiometries.

### Example 4: Pharmacokinetics

Analyses were conducted in CD-1 mice. Each animal (3/group) was injected with a single dose of each of the four ABD-Fc protein variants described above at 10mg/kg via the intraperitoneal route. Cohorts of 3 mice each for each dose were bled separately at 2 hour, 24 hours, and 72 hours, Day 5, Day 7 and Day 10, respectively. Serum procured from the bleeds was used for the determination of serum ABD-Fc levels using an anti-ABD-Fc ELISA assay. Briefly, individual wells of a 96-well Maxisorp Immunoplate (Nunc) were coated with 50 ng of a goat anti-His antibody (BD Biosciences, San Jose, CA). The plates were blocked with 5% (w/v) non-fat dry milk (Biorad), incubated with samples or standards (0.1-20 ng/ml), then with a HRP conjugate of a goat anti-mouse Fc polyclonal Ab (Pierce). Peroxidase activity was detected with 3,3',5,5'-tetramethylbenzidine (Kirkegaard & Perry Laboratories, Gaithersburg, MD), and the reaction was quenched with 0.18 M H₂SO₄. The absorbance at 450 nm was measured with a *V*ₘₐₓ kinetic microplate reader running SoftMaxPro 3.1.1 software (Molecular Devices, Sunnyvale, CA).

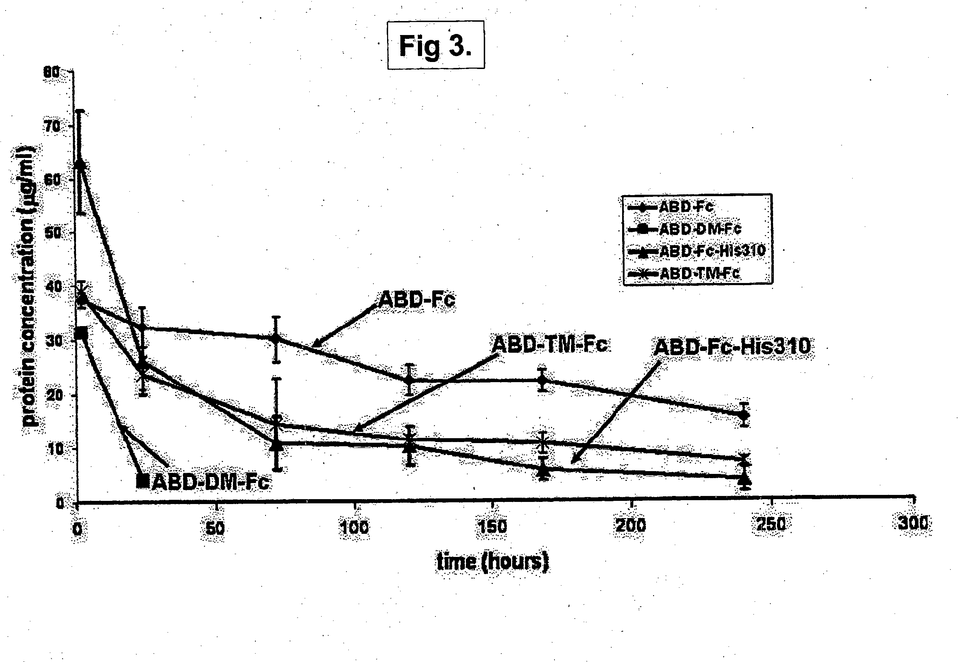

To correlate *in vitro* binding with the half-life extension, we tested all four of the ABD-Fc constructs described above in pharmacokinetic studies. All four proteins were injected into different CD-1 mice and their serum concentration (µg/ml) was determined by ELISA. Wild type ABD-Fc construct is detected at higher concentrations in the serum over a 10 day period than any of the mutant ABD-Fc variants. The ABD-TM-Fc mutant has a similar clearance rate to ABD-Fc (His310) whereas the presence of the double mutant ABD-DM could not be detected after 24 hours post-injection. See Figure 3. This data show that fusion of ABD to the Fc leads to positive synergistic effect on half-life. This also shows that combining both a serum albumin and FcRn binding IgG Fc fragment together in one molecule results in a positive synergistic effect when compared with a serum albumin or FcRn binding IgG Fc fragment alone.

## Claims

1. A molecule comprising an albumin binding domain (ABD) from Streptococcus protein G fused to an FcRn binding IgG Fc fragment, wherein the molecule comprising the ABD fused to the FcRn binding IgG Fc fragment has a longer half-life than a molecule comprising the the FcRn binding IgG Fc fragment without an ABD or comprising the ABD without the FcRn binding IgG Fc fragment.

2. The molecule of claim 1, wherein the half life is at least 10% longer.

3. The molecule of any of the preceding claims, wherein the FcRn binding IgG Fc fragment is an IgG1, IgG2, IgG3, or IgG4 Fc fragment.

4. The molecule of claim 3, wherein the Fc fragment comprises a hinge region, a CH2 domain and a CH3 domain.

5. The molecule of any of the preceding claims, wherein the ABD domain comprises an amino acid sequence of SEQ ID NO: 1; SEQ ID NO:2; or a variant of SEQ ID NO: 1 or SEQ ID NO:2.

6. The molecule of any of the preceding claims, wherein a linker peptide separates the ABD domain and the Fc fragment.

7. The molecule of any of the preceding claims, wherein the ABD domain is at the carboxyl terminal end of the molecule.

8. The molecule of any of the preceding claims, further comprising a bioactive agent of interest.

9. The molecule of any of claims 1-6, wherein the bioactive agent is a polypeptide.

10. The molecule of claim 7, wherein the polypeptide is an antibody or an antigen binding fragment thereof.

11. A composition comprising the molecule of any of the preceding claims together with a pharmaceutically acceptable carrier, adjuvant or diluent.

12. A nucleic acid sequence that encodes the molecule of any of claims 1-7.

13. A host cell comprising the nucleic acid of claim 12.

14. A method of producing a molecule, comprising culturing a cell line transfected with the nucleic acid of claim 12 and purifying the polypeptide encoded thereby.

15. A method of increasing the *in-vivo* half-life of a bioactive agent of interest comprising genetically fusing or chemically conjugating the bioactive agent with the molecule of any of claims 1-7.

## Patentansprüche

1. Molekül, umfassend eine an ein FcRn bindendes IgG-Fc-Fragment fusionierte Albumin bindende Domäne (ABD) aus Streptococcus-Protein G, wobei das die an das FcRn bindende IgG-Fc-Fragment fusionierte ABD umfassende Molekül eine längere Halbwertszeit als ein Molekül, das das FcRn bindende IgG-Fc-Fragment ohne eine ABD oder die ABD ohne das FcRn bindende IgG-Fc-Fragment umfasst, aufweist.

2. Molekül nach Anspruch 1, wobei die Halbwertszeit um wenigstens 10% länger ist.

3. Molekül nach einem der vorhergehenden Ansprüche, wobei es sich bei dem FcRn bindenden IgG-Fc-Fragment um ein IgG1-, IgG2-, IgG3- oder IgG4-Fc-Fragment handelt.

4. Molekül nach Anspruch 3, wobei das Fc-Fragment einen Scharnierbereich (Hinge), eine CH2-Domäne und eine CH3-Domäne umfasst.

5. Molekül nach einem der vorhergehenden Ansprüche, wobei die ABD-Domäne eine Aminosäuresequenz der SEQ ID NO:1; SEQ ID NO:2; oder eine Variante der SEQ ID NO:1 oder SEQ ID NO:2 umfasst.

6. Molekül nach einem der vorhergehenden Ansprüche, wobei ein Linker-Peptid die ABD-Domäne und das Fc-Fragment trennt.

7. Molekül nach einem der vorhergehenden Ansprüche, wobei sich die ABD-Domäne am carboxyterminalen Ende des Moleküls befindet.

8. Molekül nach einem der vorhergehenden Ansprüche, ferner umfassend einen interessierenden bioaktiven Wirkstoff.

9. Molekül nach einem der Ansprüche 1-6, wobei es sich bei dem bioaktiven Wirkstoff um ein Poly-peptid handelt.

10. Molekül nach Anspruch 7, wobei es sich bei dem Polypeptid um einen Antikörper oder ein antigen-bindendes Fragment davon handelt.

11. Zusammensetzung, umfassend das Molekül nach einem der vorhergehenden Ansprüche zusammen mit einem pharmazeutisch unbedenklichen Träger-, Hilfs- oder Verdünnungsmittel.

12. Nukleinsäuresequenz, die das Molekül nach einem der Ansprüche 1-7 codiert.

13. Wirtszelle, umfassend die Nukleinsäure nach Anspruch 12.

14. Verfahren zur Herstellung eines Moleküls, wobei man eine mit der Nukleinsäure nach Anspruch 12 transfizierte Zelllinie kultiviert und das davon codierte Polypeptid aufreinigt.

15. Verfahren zur Erhöhung der In-vivo-Halbwertszeit eines interessierenden bioaktiven Wirkstoffs, wobei man den bioaktiven Wirkstoff mit dem Molekül nach einem der Ansprüche 1-7 gentechnisch fusio-niert oder chemisch konjugiert.

## Revendications

1. Molécule comprenant un domaine de liaison d'albumine (ABD) de la protéine G de Streptococcus fusionné à un fragment Fc d'IgG de liaison de FcRn, la molécule comprenant l'ABD fusionné au fragment Fc d'IgG de liaison de FcRn ayant une demi-vie plus longue qu'une molécule comprenant le fragment Fc d'IgG de liaison de FcRn sans ABD ou comprenant l'ABD sans le fragment Fc d'IgG de liaison de FcRn.

2. Molécule de la revendication 1, dans laquelle la demi-vie est plus longue d'au moins 10 %.

3. Molécule de l'une quelconque des revendications précédentes, dans laquelle le fragment Fc d'IgG de liaison de FcRn est un fragment Fc d'IgG1, IgG2, IgG3, ou IgG4.

4. Molécule de la revendication 3, dans laquelle le fragment Fc comprend une région de charnière, un domaine CH2 et un domaine CH3.

5. Molécule de l'une quelconque des revendications précédentes, dans laquelle le domaine ABD comprend une séquence d'acides aminés de SEQ ID NO: 1 ; SEQ ID NO: 2 ; ou un variant de SEQ ID NO: 1 ou SEQ ID NO: 2.

6. Molécule de l'une quelconque des revendications précédentes, dans laquelle un lieur peptidique sépare le domaine ABD et le fragment Fc.

7. Molécule de l'une quelconque des revendications précédentes, dans laquelle le domaine ABD est à l'extrémité carboxyle terminale de la molécule.

8. Molécule de l'une quelconque des revendications précédentes, comprenant en outre un agent bioactif d'intérêt.

9. Molécule de l'une quelconque des revendications 1 à 6, dans laquelle l'agent bioactif est un polypeptide.

10. Molécule de la revendication 7, dans laquelle le polypeptide est un anticorps ou un fragment de liaison d'antigène de celui-ci.

11. Composition comprenant la molécule de l'une quelconque des revendications précédentes conjointement avec un véhicule, adjuvant ou diluant pharmaceutiquement acceptable.

12. Séquence d'acide nucléique qui code pour la molécule de l'une quelconque des revendications 1 à 7.

13. Cellule hôte comprenant l'acide nucléique de la revendication 12.

14. Procédé de production d'une molécule, comprenant la culture d'une lignée cellulaire transfectée avec l'acide nucléique de la revendication 12 et la purification du polypeptide codé par celui-ci.

15. Procédé d'augmentation de la demi-vie *in vivo* d'un agent bioactif d'intérêt comprenant la fusion génétique ou chimique de l'agent bioactif avec la molécule de l'une quelconque des revendications 1 à 7.


### SEQUENCE LISTING

<110> MedImmune, LLC
<120> MOLECULES WITH EXTENDED HALF-LIVES AND USES THEREOF
<130> MED0035.PCT
<150> US 61/182,858
   <151> 2009-06-01
<160> 15
<170> PatentIn version 3.5
<210> 1
   <211> 46
   <212> PRT
   <213> Streptococcus sp. G148
<400> 1
   Leu Ala Glu Ala Lys Val Leu Ala Asn Arg Glu Leu Asp Lys Tyr Gly
   1               5                   10                  15
   Val Ser Asp Tyr Tyr Lys Asn Leu Ile Asn Asn Ala Lys Thr Val Glu
               20                  25                  30
   Gly Val Lys Ala Leu Ile Asp Glu Ile Leu Ala Ala Leu Pro
           35                  40                  45
<210> 2
   <211> 45
   <212> PRT
   <213> Peptostreptococcus magnus
<400> 2
   Leu Lys Asn Ala Lys Glu Asp Ala Ile Ala Glu Leu Lys Lys Ala Gly
   1               5                   10                  15
   Ile Thr Ser Asp Phe Tyr Phe Asn Ala Ile Asn Lys Ala Lys Thr Val
               20                  25                  30
   Glu Glu Val Asn Ala Leu Lys Asn Glu Ile Leu Lys Ala
           35                  40                  45
<210> 3
   <211> 846
   <212> DNA
   <213> Artificial Sequence
<220>
   <223> Recombinant DNA
<400> 3
   gctctggctg aagctaaagt cctggctaac cgcgaactgg acaaatatgg tgtatccgac 60
   tattacaaga acctgatcaa caatgccaaa actgttgaag gtgtaaaagc actgattgat 120
   gaaattctgg ctgcactgcc tggcgccgag cccagagggc ccacaatcaa gccctgtcct 180
   ccatgcaaat gcccagcacc taacctcttg ggtggaccat ccgtcttcat cttccctcca 240
   aagatcaagg atgtactcat gatctccctg agccccatag tcacatgtgt ggtggtggat 300
   gtgagcgagg atgacccaga tgtccagatc agctggtttg tgaacaacgt ggaagtacac 360
   acagctcaga cacaaaccca tagagaggat tacaacagta ctctccgggt ggtcagtgcc 420
   ctccccatcc agcaccagga ctggatgagt ggcaaggagt tcaaatgcaa ggtcaacaac 480
   aaagacctcc cagcgcccat cgagagaacc atctcaaaac ccaaagggtc agtaagagct 540
   ccacaggtat atgtcttgcc tccaccagaa gaagagatga ctaagaaaca ggtcactctg 600
   acctgcatgg tcacagactt catgcctgaa gacatttacg tggagtggac caacaacggg 660
   aaaacagagc taaactacaa gaacactgaa ccagtcctgg actctgatgg ttcttacttc 720
   atgtacagca agctgagagt ggaaaagaag aactgggtgg aaagaaatag ctactcctgt 780
   tcagtggtcc acgagggtct gcacaatcac cacacgacta agagcttctc ccggactccg 840
   ggtaaa 846
<210> 4
   <211> 281
   <212> PRT
   <213> Artificial Sequence
<220>
   <223> Recombinant protein
<400> 4
   Leu Ala Glu Ala Lys Val Leu Ala Asn Arg Glu Leu Asp Lys Tyr Gly
   1               5                   10                  15
   Val Ser Asp Tyr Tyr Lys Asn Leu Ile Asn Asn Ala Lys Thr Val Glu
               20                  25                  30
   Gly Val Lys Ala Leu Ile Asp Glu Ile Leu Ala Ala Leu Pro Gly Ala
           35                  40                  45
   Glu Pro Arg Gly Pro Thr Ile Lys Pro Cys Pro Pro Cys Lys Cys Pro
       50                  55                  60
   Ala Pro Asn Leu Leu Gly Gly Pro Ser Val Phe Ile Phe Pro Pro Lys
   65                  70                  75                  80
   Ile Lys Asp Val Leu Met Ile Ser Leu Ser Pro Ile Val Thr Cys Val
                   85                  90                  95
   Val Val Asp Val Ser Glu Asp Asp Pro Asp Val Gln Ile Ser Trp Phe
               100                 105                 110
   Val Asn Asn Val Glu Val His Thr Ala Gln Thr Gln Thr His Arg Glu
   115 120 125
   Asp Tyr Asn Ser Thr Leu Arg Val Val Ser Ala Leu Pro Ile Gln His
   130 135 140
   Gln Asp Trp Met Ser Gly Lys Glu Phe Lys Cys Lys Val Asn Asn Lys
   145 150 155 160
   Asp Leu Pro Ala Pro Ile Glu Arg Thr Ile Ser Lys Pro Lys Gly Ser
   165 170 175
   Val Arg Ala Pro Gln Val Tyr Val Leu Pro Pro Pro Glu Glu Glu Met
   180 185 190
   Thr Lys Lys Gln Val Thr Leu Thr Cys Met Val Thr Asp Phe Met Pro
   195 200 205
   Glu Asp Ile Tyr Val Glu Trp Thr Asn Asn Gly Lys Thr Glu Leu Asn
   210 215 220
   Tyr Lys Asn Thr Glu Pro Val Leu Asp Ser Asp Gly Ser Tyr Phe Met
   225 230 235 240
   Tyr Ser Lys Leu Arg Val Glu Lys Lys Asn Trp Val Glu Arg Asn Ser
   245 250 255
   Tyr Ser Cys Ser Val Val His Glu Gly Leu His Asn His His Thr Thr
   260 265 270
   Lys Ser Phe Ser Arg Thr Pro Gly Lys
   275 280
<210> 5
   <211> 323
   <212> PRT
   <213> Artificial Sequence
<220>
   <223> Recombinant Protein
<400> 5
   Met Pro Leu Leu Leu Leu Leu Pro Leu Leu Trp Ala Gly Ala Leu Ala
   1               5                   10                  15
   Leu Ala Glu Ala Lys Val Leu Ala Asn Arg Glu Leu Asp Lys Tyr Gly
               20                  25                  30
   Val Ser Asp Tyr Tyr Lys Asn Leu Ile Asn Asn Ala Lys Thr Val Glu
           35                  40                  45
   Gly Val Lys Ala Leu Ile Asp Glu Ile Leu Ala Ala Leu Pro Gly Ala
       50                  55                  60
   Glu Pro Arg Gly Pro Thr Ile Lys Pro Cys Pro Pro Cys Lys Cys Pro
   65                  70                  75                  80
   Ala Pro Asn Leu Leu Gly Gly Pro Ser Val Phe Ile Phe Pro Pro Lys
                   85                  90                  95
   Ile Lys Asp Val Leu Met Ile Ser Leu Ser Pro Ile Val Thr Cys Val
               100                 105                 110
   Val Val Asp Val Ser Glu Asp Asp Pro Asp Val Gln Ile Ser Trp Phe
           115                 120                 125
   Val Asn Asn Val Glu Val His Thr Ala Gln Thr Gln Thr His Arg Glu
       130                 135                 140
   Asp Tyr Asn Ser Thr Leu Arg Val Val Ser Ala Leu Pro Ile Gln His
   145                 150                 155                 160
   Gln Asp Trp Met Ser Gly Lys Glu Phe Lys Cys Lys Val Asn Asn Lys
                   165                 170                 175
   Asp Leu Pro Ala Pro Ile Glu Arg Thr Ile Ser Lys Pro Lys Gly Ser
               180                 185                 190
   Val Arg Ala Pro Gln Val Tyr Val Leu Pro Pro Pro Glu Glu Glu Met
           195                 200                 205
   Thr Lys Lys Gln Val Thr Leu Thr Cys Met Val Thr Asp Phe Met Pro
       210                 215                 220
   Glu Asp Ile Tyr Val Glu Trp Thr Asn Asn Gly Lys Thr Glu Leu Asn
   225                 230                 235                 240
   Tyr Lys Asn Thr Glu Pro Val Leu Asp Ser Asp Gly Ser Tyr Phe Met
                   245                 250                 255
   Tyr Ser Lys Leu Arg Val Glu Lys Lys Asn Trp Val Glu Arg Asn Ser
               260                 265                 270
   Tyr Ser Cys Ser Val Val His Glu Gly Leu His Asn His His Thr Thr
           275                 280                 285
   Lys Ser Phe Ser Arg Thr Pro Gly Lys Gly Gly Gly Gly Ser Glu Gln
       290                 295                 300
   Lys Leu Ile Ser Glu Glu Asp Leu Gly Gly Gly Gly Ser His His His
   305 310 315 320
   His His His
<210> 6
   <211> 323
   <212> PRT
   <213> Artificial Sequence
<220>
   <223> Recombinant Protein
<400> 6
   Met Pro Leu Leu Leu Leu Leu Pro Leu Leu Trp Ala Gly Ala Leu Ala
   1               5                   10                  15
   Leu Ala Glu Ala Lys Val Leu Ala Asn Arg Glu Leu Asp Lys Tyr Gly
               20                  25                  30
   Val Ala Asp Ala Tyr Ala Asn Leu Ile Asn Asn Ala Lys Thr Val Glu
           35                  40                  45
   Gly Val Lys Ala Leu Ile Asp Glu Ile Leu Ala Ala Leu Pro Gly Ala
       50                  55                  60
   Glu Pro Arg Gly Pro Thr Ile Lys Pro Cys Pro Pro Cys Lys Cys Pro
   65                  70                  75                  80
   Ala Pro Asn Leu Leu Gly Gly Pro Ser Val Phe Ile Phe Pro Pro Lys
                   85                  90                  95
   Ile Lys Asp Val Leu Met Ile Ser Leu Ser Pro Ile Val Thr Cys Val
               100                 105                 110
   Val Val Asp Val Ser Glu Asp Asp Pro Asp Val Gln Ile Ser Trp Phe
           115                 120                 125
   Val Asn Asn Val Glu Val His Thr Ala Gln Thr Gln Thr His Arg Glu
       130                 135                 140
   Asp Tyr Asn Ser Thr Leu Arg Val Val Ser Ala Leu Pro Ile Gln His
   145                 150                 155                 160
   Gln Asp Trp Met Ser Gly Lys Glu Phe Lys Cys Lys Val Asn Asn Lys
                   165                 170                 175
   Asp Leu Pro Ala Pro Ile Glu Arg Thr Ile Ser Lys Pro Lys Gly Ser
               180                 185                 190
   Val Arg Ala Pro Gln Val Tyr Val Leu Pro Pro Pro Glu Glu Glu Met
           195                 200                 205
   Thr Lys Lys Gln Val Thr Leu Thr Cys Met Val Thr Asp Phe Met Pro
       210                 215                 220
   Glu Asp Ile Tyr Val Glu Trp Thr Asn Asn Gly Lys Thr Glu Leu Asn
   225                 230                 235                 240
   Tyr Lys Asn Thr Glu Pro Val Leu Asp Ser Asp Gly Ser Tyr Phe Met
                   245                 250                 255
   Tyr Ser Lys Leu Arg Val Glu Lys Lys Asn Trp Val Glu Arg Asn Ser
               260                 265                 270
   Tyr Ser Cys Ser Val Val His Glu Gly Leu His Asn His His Thr Thr
           275                 280                 285
   Lys Ser Phe Ser Arg Thr Pro Gly Lys Gly Gly Gly Gly Ser Glu Gln
       290                 295                 300
   Lys Leu Ile Ser Glu Glu Asp Leu Gly Gly Gly Gly Ser His His His
   305                 310                 315                 320
   His His His
<210> 7
   <211> 323
   <212> PRT
   <213> Artificial Sequence
<220>
   <223> Recombinant Protein
<400> 7
   Met Pro Leu Leu Leu Leu Leu Pro Leu Leu Trp Ala Gly Ala Leu Ala
   1               5                   10                  15
   Leu Ala Glu Ala Lys Val Leu Ala Asn Arg Glu Leu Asp Lys Tyr Gly
               20                  25                  30
   Val Ser Asp Tyr Tyr Lys Asn Leu Ile Asn Asn Ala Lys Thr Val Glu
           35                  40                  45
   Gly Val Lys Ala Leu Ile Asp Glu Ile Leu Ala Ala Leu Pro Gly Ala
       50                  55                  60
   Glu Pro Arg Gly Pro Thr Ile Lys Pro Cys Pro Pro Cys Lys Cys Pro
   65                  70                  75                  80
   Ala Pro Asn Leu Leu Gly Gly Pro Ser Val Phe Ile Phe Pro Pro Lys
                   85                  90                  95
   Ile Lys Asp Val Leu Met Ile Ser Leu Ser Pro Ile Val Thr Cys Val
               100                 105                 110
   Val Val Asp Val Ser Glu Asp Asp Pro Asp Val Gln Ile Ser Trp Phe
           115                 120                 125
   Val Asn Asn Val Glu Val His Thr Ala Gln Thr Gln Thr His Arg Glu
       130                 135                 140
   Asp Tyr Asn Ser Thr Leu Arg Val Val Ser Ala Leu Pro Ile Gln Ala
   145                 150                 155                 160
   Gln Asp Trp Met Ser Gly Lys Glu Phe Lys Cys Lys Val Asn Asn Lys
                   165                 170                 175
   Asp Leu Pro Ala Pro Ile Glu Arg Thr Ile Ser Lys Pro Lys Gly Ser
               180                 185                 190
   Val Arg Ala Pro Gln Val Tyr Val Leu Pro Pro Pro Glu Glu Glu Met
           195                 200                 205
   Thr Lys Lys Gln Val Thr Leu Thr Cys Met Val Thr Asp Phe Met Pro
       210                 215                 220
   Glu Asp Ile Tyr Val Glu Trp Thr Asn Asn Gly Lys Thr Glu Leu Asn
   225                 230                 235                 240
   Tyr Lys Asn Thr Glu Pro Val Leu Asp Ser Asp Gly Ser Tyr Phe Met
                   245                 250                 255
   Tyr Ser Lys Leu Arg Val Glu Lys Lys Asn Trp Val Glu Arg Asn Ser
               260                 265                 270
   Tyr Ser Cys Ser Val Val His Glu Gly Leu His Asn His His Thr Thr
           275                 280                 285
   Lys Ser Phe Ser Arg Thr Pro Gly Lys Gly Gly Gly Gly Ser Glu Gln
       290                 295                 300
   Lys Leu Ile Ser Glu Glu Asp Leu Gly Gly Gly Gly Ser His His His
   305                 310                 315                 320
   His His His
<210> 8
   <211> 232
   <212> PRT
   <213> Homo sapiens
<400> 8
   Glu Pro Lys Ser Cys Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Glu Glu Met Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 9
   <211> 228
   <212> PRT
   <213> Homo sapiens
<400> 9
   Glu Arg Lys Cys Cys Val Glu Cys Pro Pro Cys Pro Ala Pro Pro Val
   1               5                   10                  15
   Ala Gly Pro Ser Val Phe Leu Phe Pro Pro Lys Pro Lys Asp Thr Leu
               20                  25                  30
   Met Ile Ser Arg Thr Pro Glu Val Thr Cys Val Val Val Asp Val Ser
           35                  40                  45
   His Glu Asp Pro Glu Val Gln Phe Asn Trp Tyr Val Asp Gly Val Glu
       50                  55                  60
   Val His Asn Ala Lys Thr Lys Pro Arg Glu Glu Gln Phe Asn Ser Thr
   65                  70                  75                  80
   Phe Arg Val Val Ser Val Leu Thr Val Val His Gln Asp Trp Leu Asn
                   85                  90                  95
   Gly Lys Glu Tyr Lys Cys Lys Val Ser Asn Lys Gly Leu Pro Ala Pro
               100                 105                 110
   Ile Glu Lys Thr Ile Ser Lys Thr Lys Gly Gln Pro Arg Glu Pro Gln
           115                 120                 125
   Val Tyr Thr Leu Pro Pro Ser Arg Glu Glu Met Thr Lys Asn Gln Val
       130                 135                 140
   Ser Leu Thr Cys Leu Val Lys Gly Phe Tyr Pro Ser Asp Ile Ser Val
   145                 150                 155                 160
   Glu Trp Glu Ser Asn Gly Gln Pro Glu Asn Asn Tyr Lys Thr Thr Pro
                   165                 170                 175
   Pro Met Leu Asp Ser Asp Gly Ser Phe Phe Leu Tyr Ser Lys Leu Thr
               180                 185                 190
   Val Asp Lys Ser Arg Trp Gln Gln Gly Asn Val Phe Ser Cys Ser Val
           195                 200                 205
   Met His Glu Ala Leu His Asn His Tyr Thr Gln Lys Ser Leu Ser Leu
       210                 215                 220
   Ser Pro Gly Lys
   225
<210> 10
   <211> 279
   <212> PRT
   <213> Homo sapiens
<400> 10
   Glu Leu Lys Thr Pro Leu Gly Asp Thr Thr His Thr Cys Pro Arg Cys
   1 5 10 15
   Pro Glu Pro Lys Ser Cys Asp Thr Pro Pro Pro Cys Pro Arg Cys Pro
   20 25 30
   Glu Pro Lys Ser Cys Asp Thr Pro Pro Pro Cys Pro Arg Cys Pro Glu
   35 40 45
   Pro Lys Ser Cys Asp Thr Pro Pro Pro Cys Pro Arg Cys Pro Ala Pro
   50 55 60
   Glu Leu Leu Gly Gly Pro Ser Val Phe Leu Phe Pro Pro Lys Pro Lys
   65 70 75 80
   Asp Thr Leu Met Ile Ser Arg Thr Pro Glu Val Thr Cys Val Val Val
   85 90 95
   Asp Val Ser His Glu Asp Pro Glu Val Gln Phe Lys Trp Tyr Val Asp
   100 105 110
   Gly Val Glu Val His Asn Ala Lys Thr Lys Pro Arg Glu Glu Gln Tyr
   115 120 125
   Asn Ser Thr Phe Arg Val Val Ser Val Leu Thr Val Leu His Gln Asp
   130 135 140
   Trp Leu Asn Gly Lys Glu Tyr Lys Cys Lys Val Ser Asn Lys Ala Leu
   145 150 155 160
   Pro Ala Pro Ile Glu Lys Thr Ile Ser Lys Thr Lys Gly Gln Pro Arg
   165 170 175
   Glu Pro Gln Val Tyr Thr Leu Pro Pro Ser Arg Glu Glu Met Thr Lys
   180 185 190
   Asn Gln Val Ser Leu Thr Cys Leu Val Lys Gly Phe Tyr Pro Ser Asp
   195 200 205
   Ile Ala Val Glu Trp Glu Ser Ser Gly Gln Pro Glu Asn Asn Tyr Asn
       210                 215                 220
   Thr Thr Pro Pro Met Leu Asp Ser Asp Gly Ser Phe Phe Leu Tyr Ser
   225                 230                 235                 240
   Lys Leu Thr Val Asp Lys Ser Arg Trp Gln Gln Gly Asn Ile Phe Ser
                   245                 250                 255
   Cys Ser Val Met His Glu Ala Leu His Asn Arg Phe Thr Gln Lys Ser
               260                 265                 270
   Leu Ser Leu Ser Pro Gly Lys
           275
<210> 11
   <211> 229
   <212> PRT
   <213> Homo sapiens
<400> 11
   Glu Ser Lys Tyr Gly Pro Pro Cys Pro Ser Cys Pro Ala Pro Glu Phe
   1               5                   10                  15
   Leu Gly Gly Pro Ser Val Phe Leu Phe Pro Pro Lys Pro Lys Asp Thr
               20                  25                  30
   Leu Met Ile Ser Arg Thr Pro Glu Val Thr Cys Val Val Val Asp Val
           35                  40                  45
   Ser Gln Glu Asp Pro Glu Val Gln Phe Asn Trp Tyr Val Asp Gly Val
       50                  55                  60
   Glu Val His Asn Ala Lys Thr Lys Pro Arg Glu Glu Gln Phe Asn Ser
   65                  70                  75                  80
   Thr Tyr Arg Val Val Ser Val Leu Thr Val Leu His Gln Asp Trp Leu
                   85                  90                  95
   Asn Gly Lys Glu Tyr Lys Cys Lys Val Ser Asn Lys Gly Leu Pro Ser
               100                 105                 110
   Ser Ile Glu Lys Thr Ile Ser Lys Ala Lys Gly Gln Pro Arg Glu Pro
           115                 120                 125
   Gln Val Tyr Thr Leu Pro Pro Ser Gln Glu Glu Met Thr Lys Asn Gln
       130                 135                 140
   Val Ser Leu Thr Cys Leu Val Lys Gly Phe Tyr Pro Ser Asp Ile Ala
   145                 150                 155                 160
   Val Glu Trp Glu Ser Asn Gly Gln Pro Glu Asn Asn Tyr Lys Thr Thr
                   165                 170                 175
   Pro Pro Val Leu Asp Ser Asp Gly Ser Phe Phe Leu Tyr Ser Arg Leu
               180                 185                 190
   Thr Val Asp Lys Ser Arg Trp Gln Glu Gly Asn Val Phe Ser Cys Ser
           195                 200                 205
   Val Met His Glu Ala Leu His Asn His Tyr Thr Gln Lys Ser Leu Ser
       210                 215                 220
   Leu Ser Leu Gly Lys
   225
<210> 12
   <211> 8
   <212> PRT
   <213> Homo sapiens
<400> 12
   His Gln Asn Leu Ser Asp Gly Lys
   1 5
<210> 13
   <211> 8
   <212> PRT
   <213> Homo sapiens
<400> 13
   His Gln Asn Ile Ser Asp Gly Lys
   1 5
<210> 14
   <211> 8
   <212> PRT
   <213> Homo sapiens
<400> 14
   Val Ile Ser Ser His Leu Gly Gln
   1 5
<210> 15
   <211> 11
   <212> PRT
   <213> Homo sapiens
<400> 15
   Pro Lys Asn Ser Ser Met Ile Ser Asn Thr Pro
   1 5 10